(19) Europäisches Patentamt
European Patent Office
Office européen des brevets

(11) EP 0 533 849 B1

(12) **EUROPEAN PATENT SPECIFICATION**

(45) Date of publication and mention of the grant of the patent:
**22.07.1998 Bulletin 1998/30**

(21) Application number: **91918781.5**

(22) Date of filing: **28.10.1991**

(51) Int Cl.[6]: **C12N 15/54**, C12N 9/10, C07K 14/00, C12N 15/82, C12P 21/08, C12N 5/20, A01H 5/00

(86) International application number:
**PCT/EP91/02034**

(87) International publication number:
**WO 92/16631 (01.10.1992 Gazette 1992/25)**

(54) **SUCROSE PHOSPHATE SYNTHASE (SPS), ITS PROCESS FOR PREPARATION, ITS cDNA, AND UTILIZATION OF cDNA TO MODIFY THE EXPRESSION OF SPS IN THE PLANT CELLS**

SUKROSEPHOSPHATSYNTHASE (SPS), VERFAHREN ZU IHRER HERSTELLUNG, IHRE C-DNS, UND VERWENDUNG DER C-DNS ZUR MODIFIZIERUNG DER SPS-EXPRESSION IN PFLANZENZELLEN

SYNTHASE DE PHOSPHATE DE SUCROSE (SPS), PROCEDE DE PREPARATION, SON ADNc ET UTILISATION DE L'ADNc POUR MODIFIER L'EXPRESSION DE SPS DANS LES CELLULES VEGETALES

(84) Designated Contracting States:
**AT BE CH DE DK ES FR GB GR IT LI LU NL SE**

(30) Priority: **18.03.1991 US 672646**

(43) Date of publication of application:
**31.03.1993 Bulletin 1993/13**

(60) Divisional application: **96200003.0 / 0 718 401**

(73) Proprietor: **ROUSSEL UCLAF**
**93230 Romainville (FR)**

(72) Inventors:
- **VAN ASSCHE, Charles, La Tartane**
  **F-13009 Marseille (FR)**
- **LANDO, Danielle**
  **F-75020 Paris (FR)**
- **BRUNEAU, Jean-Michel**
  **F-75011 Paris (FR)**
- **VOELKER, Toni, Alois**
  **Davis, CA 95616 (US)**
- **GERVAIS, Monica**
  **F-95320 Saint-Leu-La-Forêt (FR)**

(74) Representative: **Vieillefosse, Jean-Claude et al**
**Hoechst Marion Roussel**
**Département des Brevets**
**102, Route de Noisy**
**93235 Romainville Cédex (FR)**

(56) References cited:
**EP-A- 438 904**

- **JOURNAL OF CELLULAR BIOCHEMISTRY, suppl. 15A, January 1991, New York, NY (US); T.A. VOELKER et al., p. 84, AN A-361**
- **PHYSIOLOGY PLANTARIUM, vol. 70, 1987, Copenhagen (DK); W. KALT-TORRES et al.,**
- **pp. 653-658**
- **PLANTA, vol. 170, 1987, Berlin (DE); P.S. KERR et al., pp. 515-519**
- **PLANT PHYSIOLOGY, vol. 96, no. 2, June 1991, American Society of Plant Physiologists, Rockville, MD (US); J.-M. BRUNEAU et al., pp. 473-478**
- **THE PLANT CELL, vol. 3, October 1991, American Society of Plant Physiologists, Rockville, MD (US); A.C. WORRELL et al., pp. 1121-1130**
- **BIOTECHNOLOGY, vol. 5, July 1987, New York, NY (US); J.J. FILLATTI et al., pp. 726-730**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

- PLANT MOLECULAR BIOLOGY, vol. 14, no. 2, February 1990, Nijhoff Publishers, Dordrecht (NL); K.E. McBRIDE et al., pp. 269-276
- PHYSIOLOGIA PLANTARIUM, vol. 77, 1989, Copenhagen (DK); M. STITT et al., pp. 633-641

## Description

Difficulties in the purification of sucrose phosphate synthase (SPS) from plants have interferred with efforts to characterize this enzyme. SPS catalyses the formation of sucrose phosphate, the sucrose precursor molecule, from fructose-6 phosphate and UDP-glucose in photosynthetically active plant cells. Sucrose phosphatase then acts on the sucrose phosphate moiety, in an irreversible reaction, to remove the phosphate and to release sucrose ready to translocate from the mature leaf (source) to any tissue requiring photoassimilate (sink), especially growing tissues including young leaves, seeds, and roots.

Because SPS is considered a rate limiting enzyme in the pathway providing sucrose to growing tissue, the study of SPS and its activity is of special interest. In a recent publication, Walker, J. L. & Huber, S.C., Plant Phys. (1989) 89 : 518-524, the purification and preliminary characterization of spinach (Spinachia oleracea) SPS was reported. However, monoclonal antibodies specific to the spinach SPS were found to be non-reactive with all other plants tested, "closely related" and "relatively unrelated species", including corn (Zea maize), soybean (Glycine max), barley (Hordeum vulgare), and sugar beet (Beta vulgaris). Thus, additional purified sources of SPS enzyme are needed for effective characterization of this factor. Especially of interest is the characterization of the corn SPS because of its very high export rates, as compared for example, to SPS levels of activity as found in the leaves of soybean.

With the advent of biotechnology, the ability to modify various properties of plants, especially agronomically important crops, is of interest. In this regard, it would be useful to determine the coding sequence for an SPS gene to probe other crop sources, to use such coding sequences to prepare DNA expression constructs capable of directing the expression of the SPS gene in a plant cell and to express a DNA sequence encoding an SPS enzyme in a plant to measure the effects on crop yield due to the increased rate of sucrose translocation to growing tissues.

In a first embodiment, homogenous protein being capable of catalyzing the formation of sucrose phosphate wherein said proteins obtainable from corn protein synthase (SPS) activity, namely, a protein capable of catalyzing the formation of sucrose phosphate (SPS) having fructose-6-phosphate and UDP-glucose substrates, are provided.

By "protein" is meant any amino acid sequence, including a protein, polypeptide, or peptide fragment, whether obtained from plant or synthetic sources, which demonstrates the ability to catalyze the formation of sucrose phosphate. An SPS of this invention will include sequences which are modified, such as sequence which have been mutated, trucated, increased, contain codon substitutions as a result of the degeneracy of the DNA code, and the like as well as sequences which are partially or wholly artificially synthesized, so long as the synthetic sequence retains the characteristic SPS activity.

By "homogenous protein" is meant that the protein has been partially purified away from proteins found in the plant cell. Such a protein of this invention will demonstrate a specific enzymatic activity of at least greater than 0,05, more preferably at least greater than at least 0,30, wherein specific enzymatic activity (sA) is measured in units which correspond to 1 µm (micromole) of sucrose formed per minute per mg of protein at 37°C. In a more preferred embodiment, the protein will demonstrate even more improved sA and increased purification factors (see, Table 1).

The invention relates to the protein comprising a corn SPS having a molecular weight from 110 to 130 kilodalton (kd) and a specific activity of greater than 0,05 U.

The invention relates more particularly to the protein comprising a corn SPS having a specific activity of 25 U.

In order to obtain the nucleic acid sequences encoding the SPS, especially corn SPS, substantially purified SPS was required. As demonstrated more fully in the examples, corn SPS purified 500-fold was obtained in small quantities which were then ultimately used to obtain peptide sequence which in turn led to the determination of the cDNA sequence.

Among the preferred proteins of the invention are the proteins having the above definition with a molecular weight from about 110 to about 130 kd, having the form of a monomer, a dimer or a tetramer and their derivatives, comprising at least one peptide having the following amino acid sequence :

```
Thr-Trp-Ile-Lys
Tyr-Val-Val-Glu-Leu-Ala-Arg
Ser-Met-Pro-Pro-Ile-Trp-Ala-Glu-Val-Met-Arg
Leu-Arg-Pro-Asp-Gln-Asp-Tyr-Leu-Met-His-Ile-Ser-His-Arg
Trp-Ser-His-Asp-Gly-Ala-Arg
```

The invention also relates to a process to prepare proteins as above defined, characterized in that :

**a)** one extracts from parts containing SPS, preserved at low temperature, by grinding, centrifugation and filtration,

**b)** one increases the SPS rate of the extract so obtained by precipitation in an appropriate solvent, centrifugation and solubilization of the precipitate in a buffer solution.
**c)** one purifies the protein so obtained by chromatography and if desired,
**d)** one prepares the hybridomas, and monoclonal antibodies from an antigenic solution prepared from one of the preparations a, b, c,
**e)** one screens the hybridomas and raises monoclonal antibodies specifically directed against SPS,
**f)** one purifies the SPS so obtained with the monoclonal antibodies so prepared.

The invention more precisely relates to a process of preparation of corn SPS characterized in that :

**a)** one extracts from part of corn plants by grinding centrifugation and filtration,
**b)** one increases the SPS rate of the extract so obtained by precipitation in polyethyleneglycol (PEG), centrifugation and solubilization of the precipitate obtained in a buffer solution,
**c)** one purifies the protein obtained in low pressure anion exchange chromatography and in chromatography on heparin sepharose, then in anion exchange high performance chromatography,
**d)** one purifies the active pools by passage on 2 high performance chromatography columns, and if desired,
**e)** one prepares the hybridomas and monoclonal antibodies from an antigenic solution prepared from one preparation a, b, c,
**f)** one screens the hybridomas and raises the monoclonal antibodies specifically directed against SPS,
**g)** one purifies the SPS preparation with the monoclonal antibodies so obtained.

Preferably :

- corn is a corn Pioneer corn hybrid strain 3184,
- part of plants are leaves kept at low temperature by example between -50°C and -90°C,
- purification in the polyethyleneglycol is realized

  . first by precipitating at a final concentration in PEG about 6 %,
  . then by precipitating at a final concentration about 12 %. The different chromatographies are realized in the following way :

    1st chromatography DEAE sepharose
    2nd chromatography heparin sepharose : at this stage, the preparation obtained may be kept several days without loss of activity
    3rd chromatography Mono q chromatography
    4th chromatography HPLC hydroxyapatite
    5th chromatography HPLC hydroxyapatite.

- during the different steps of purification and thereafter, the SPS activity may be measured according two methods,

  **a)** a method based on a colorimetric test or resorcinol test,
  **b)** a method based on the dosage of one of the products formed during the transformation reactions where SPS is involved.

Both methods are detailed in the experimental part detailed hereunder.
Mice are immunized with several injections of enzymatic preparations.
Different kinds of mice may be used, for example BALB/c.
The antigen may be used in completed Freund adjuvant then in incompleted Freund adjuvant. Several injections in mice are realized : good results have been obtained with three injections of Mono q, pools, followed by three injections of final pools (days 0, 14, 27, 60, 90 and 105 for example).
The first injections are administered sub-cutaneously, for example in the cushions, and the feet, the last injection is administered intravenously in the tail for example.

- the preparation of spleen cellular suspensions so immunized is made in a conventional way.

The steps of fusion with myelona cells, of conservation of the hybridoma, of cloning, of antibodies production are made by conventional ways.
To detect the hybridoma secreting the monoclonal antibodies raised against the antigen, two methods are used

to select antibodies :

- a method of detection of antibodies as inhibitor of SPS activity,
- a method of detection of antibodies precipitating SPS activities.

In a preferred embodiment, these methods are the methods described in the experimental section detailed hereunder.

Among the objects of the invention, are also provided lines of hybridoma cells, and in particular hybridoma cells described as :

| | |
|---|---|
| SPA 2-2-3 : I-971 | SPB 3-2-19 : I-973 |
| SPA 2-2-22 : I-970 | SPB 5-2-10 : I-974 |
| SPA 2-2-25 : I-972 | SPB 5-4-2 : I-975 |
| | SPB 13-1-7 : I-976 |
| | SPB 13-2-2 : I-977 |

a deposit of which has been made at the C.N.C.M. ( INSTITUT PASTEUR PARIS ) on JUNE 11,1990.

The invention relates also to monoclonal antibodies specifically directed against SPS.

The invention relates also to a process of preparation of proteins as defined above characterized in that a preparation containing the socalled proteins is purified on a chromatography column having monoclonal antibodies as defined above specifically raised against the proteins.

The invention relates also to cDNA coding for proteins as defined above, specially cDNA coding for corn SPS. Among the preferred cDNA preferred is the cDNA with the following nucleotide sequence represented in Figure 7.

Thus, this invention relates to an extrachromosomal DNA sequence encoding a SPS as defined above. Any DNA sequence which is not incorporated into the genome of a plant is considered extrachromosomal, i.e., outside of the chromosome, for purposes of this invention. This includes, but is not limited to cDNA, genomic DNA, truncated sequences, single stranded and double stranded DNA. In a preferred embodiment, the DNA sequence is cDNA. In a different preferred embodiment, the DNA sequence is obtainable from corn.

Among the preferred proteins and nucleic acid sequences of the invention is corn SPS. The corn SPS is represented in Figure 1, which shows the presents of proteins at about 120, 95 and 30 kd. The proteins shown at 95 and 30 kd are considered to be breakdown products of the protein shown at 120 kd. The complete protein is believed to a a di- or tetrameric protein having as the basic sub-unit from about a 110 to about 130 kd protein. The complete cDNA sequence of the corn SPS is shown in Figure 7.

cDNA coding for sucrose phosphate synthase has been prepared in the following way.

1) Sequencing of peptide fragments from purified SPS. With the purified preparations of SPS previously obtained, by separating on acrylamide gel, a 120 kd minor band (corresponding to the total protein sequence) and two 90 kd and 30 kd major bands are obtained. Both major polypeptides are separated on electrophoresis and electroeluted. By a trypsin digestion and the sequencing of fragments so obtained, the sequence of 5 peptides has been determined.

This aminoacid sequence allows to determine the corresponding degenerate nucleotide sequence.

2) Corn leaf isolation.

Total RNA is isolated according to TURPEN and GRIFFITH (1986, Biotechniques vol. 4 pages 11-15) for poly (A) RNA preparation, the standard oligo dT cellulose column was used.

3) cDNA library construction.

cDNA synthesis is realized by following the protocol of a kit supplied by PROMEGA except that M-MLV reverse transcriptase is used instead of AMV reverse transcriptase. The length of cDNA obtained is from 500 to several thousand base pairs. One adds ECORI linkers to the blunt ended cDNA and clones this material into a second generation lambda GT11 expression vector. Total library size is about $1{,}5.10^6$ plaques.

4) Utilization of PCR in order to synthesize a nucleotide sequence specific for SPS.

The oligonucleotides derived from peptides B11 (SPS 30 kd) and 4K (90 kd) described in figure 3 are used as primers in a PCR reaction. It has been assumed that peptides derived from SPS 30 and SPS 90 are degradation products of protein SPS 120 kd, and that, peptides derived from SPS and SPS 90 are encoded by the same RNA.

With this hypothesis, by using in proper polarity pairs of oligonucleotides corresponding to the peptidic sequences in a PCR reaction, one may obtain the synthesis of the DNA, connecting the two location. Since it is a priori not know in which order the peptides are located relative to each other, one has to do the two different possibilites Fig. 4. Only the oligonucleotide couple CD3 synthesizes a cDNA of defined length (1200 bp) (Fig. 5).

5) cDNA libray screening.

When 250000 lambda clones GTII are screened using the 1200 bp long PCR cDNA, 16 positives are obtained. Sizes of the inserts ranged from 0,3 kb to 2,8 kb (see Fig. 6 for the two longest clones). The sequence is not complete in 5'. In a second round of library screening with a 400 bp DNA fragment corresponding to the most 5' fragment of the clone SPS 3, we obtain a SPS 61 clone extending further 5' without having the 5' end of the reading frame (Fig. 6).

6) Creation and screening of a second cDNA libray in order to clone the 5' sequence of cDNA coding for SPS.

A oligonucleotide complementary to the 5' sequence of clone SPS 61 is used as a primer for cDNA synthesis. After second strand reaction is completed, the cDNA is cloned into bacteriophage lambda GT11. The library includes about one million clones. The SPS 90 and SP 77 have been obtained, by screening this library with SPS 61 (Fig. 6).

7) The assembled SPS reading frame.

DNA sequences which encode the SPS may be employed as a gene of interest in a DNA construct or as probes in accordance with this invention. When found in a hoste cell, the sequence may be expressed as a source of SPS. More preferred is the SPS sequence in a vegetal cell under the regulating control of transcriptional and translational initiation region functional in plants.

Vegetal cell means any plant cell being able to form undifferentiated tissues as callus or differentiated tissues as embryos, parts of plants, whole plants or seeds.

Plants means for example plant producing grain seeds for example such as cereals, such as wheat, barley, corn, or oat, leguminous such as soybean, oleaginous as turnesol, tuber as potato, plan with roots as beet or fruit as tomato. The sucrose phosphate synthase is a key enzyme, in sucrose regulation mechanisms, but also in carbon partitioning regulation between starch and sucrose during photosynthesis (see Jack PREISS, TIBS January 1984, page 24, or Mark STITT and Coll, BIOCHEMISTRY of PLANTS, vol. 10, 1987, pages 3-27).

When found in a DNA construct for integration into a plant genome, the sequence may be found is a sense orientation or anti-sense orientation. By increasing the amount of SPS available to the photosynthetically active plant cell by the expression of additional SPS, an increased flow of sucrose may be provided to growing tissues, for example, resulting in increased plant yields ; by decreasing the amount of SPS available to the photosynthetically active plant cell, the rate of sucrose release from the plant cell may be hindered, resulting in less new plant growth.

By "obtainable from corn" is meant that the sequence, whether an amino acid sequence or nucleic acide sequence, is related to a corn SPS, including a SPS recovered through use of nucleic acid probes, antibody preparations, sequence comparisons or derivatives obtained through protein modeling or mutagenesis for example. Thus, one skilled in the art will readily recognize that antibody preparation, nucleic acid probes (DNA and RNA) and the like may be prepared and used to screen and recover other plant sources for SPS. Typically, a homologously related nucleic acid sequence will show at least about 60 % homology, and more preferably at least about 70 % homology between the corn SPS and the given plant SPS of interest, excluding any deletions which may be present. Homology is found when there is an identity of base pairs an may be determined upon comparison of sequence information, nucleic acid or amino acide, or through hybridization reactions conducted under relatively stringent conditions, e.g., having a fairly low percentage of non-specific binding with corn SPS probes.

Probes can be considerably shorter than the entire sequence, but should be at least about 10, preferably at least about 15, more preferably at least 20 nucleotides in length. Longer oligonucleotides are also useful, up to full length of the gene encoding the polypeptide of interest. Both DNA and RNA probes can be used.

A genomic library prepared from the plant source of interest may be probed with conserved sequences from corn SPS to identify homologously related sequences. Use of the entire corn SPS cDNA may be employed if shorter probe sequences are not identified. Positive clones are then analyzed by restriction enzyme digestion and/or sequencing. In this general manner, one or more sequences may be identified providing both the coding region, as well as the transcriptional regulatory elements of the SPS gene from such plant source.

In use, probes are typically labeled in a detectable manner (for example with $^{32}P$-labelled or biotinylated nucleotides) and are incubated with single-stranded DNA or RNA from the plant source in which the gene is sought, although unlabeled oligonucleotides are also useful. Hybridization is detected by means of the label after single-stranded and double-stranded (hybridized) DNA or DNA/RNA have been separated, typically using nitrocellulose paper or nylon membranes. Hybridization techniques suitable for use with oligonucleotides are well known to those skilled in the art.

From cDNA sequences, one skilled in the art will be readily able to obtain the corresponding genomic DNA sequences related thereto to obtain the coding region of the SPS including intron sequences, transcription, translation initiation regions and/or transcript termination regions of the respective SPS gene. The regulatory regions may be used with or without the SPS gene in various probes and/or constructs.

The complete SPS reading frame can be assembled using restriction enzyme fragments of SPS 90, SPS 61 and SPS 3, see Fig. 6.

When expressed in E. Coli, the SPS cDNA produces a protein which is recognized by anti SPS antisera and has the same electrophoretic mobility as SPS extracted from corn leaves. We show that this E. Coli SPS is as active as plant SPS, i.e. for complete enzymatic activity in E. Coli no other plant factor is needed but the SPS cDNA.

Plants obtained by the method of transformation and contain fusions of SPS cDNA to tissue specific promoters in order to modify or alter the composition of certain plant organs is also included.

A DNA construct of this invention may include transcriptional and translational initiation regulatory regions homologous or heterologous to the plant host. Of particular interest are transcriptional initiation regions from genes which are present in the plant host species, for example, the tobacco ribulose biphosphate carboxylase small subunit (ssu) transcriptional initiation region ; the cauliflower mosaic virus (CaMV) 35S transcriptional initiation region, including a "double" 35S CaMV promoter ; and those associated with T-DNA, such as the opine synthase transcriptional initiation region, e.g., octopine, mannopine, agropine, and the like.

Any one of number of regulatory sequences may be preferred in a particular situation, depending upon whether constitutive or tissue and or timing induced transcription is desired, the particular efficiency of the promoter in conjunction with the heterologous SPS, the ability to join a strong promoter with a control region from a different promoter which allows of inducible transcription, ease of construction and the like. For example, tissue specific promoters may be employed to selectively modify or alter the composition of certain plant organs. These regulatory regions find ample precedence in the literature.

The termination region may be derived from the 3'-region of the gene from which the initiation region was obtained, from the SPS gene, or from a different gene. Preferably the termination region will be derived from a plant gene, particularly, the tobacco ribulose biphosphate carboxylase small subunit termination region ; a gene associated with the Ti-plasmid such as the octopine synthase termination region or the tml termination region.

In developing the expression cassette, the various fragments comprising the regulatory regions and open reading frame may be subjected to different processing conditions, such a ligation, restriction, resection, in vitro mutagenesis, primer repair, use of linkers and adapters, and the like. Thus, nucleotide transitions, transversions, insertions, deletions, or the like, may be performed on the DNA which is employed in the regulatory regions and/or open reading frame.

During the construction of the expression cassette, the various fragments of the DNA will usually be cloned in an appropriate cloning vector, which allows for amplification of the DNA, modification of the DNA or manipulation by joining or removing of the sequences, linkers, or the like. Normally, the vectors will be capable of replication in at least a relatively high copy number in E. Coli. A number of vectors are readily available for cloning, including such vectors as pBR322, pUC series, M13 series, etc. The cloning vector will have one or more markers which provide for selection or transformants. The markers will normally provide for resistance to cytotoxic agents such as antibiotics, heavy metals, toxins, or the like. By appropriate restriction of the vector and cassette, and as appropriate, modification of the ends, by chewing back or filling in overhangs, to provide for blunt ends, by addition of linkers, by tailing, complementary ends can be provided for ligation and joining of the vector to the expression cassette or component thereof.

After each manipulation of the DNA in the development of the cassette, the plasmid will be cloned and isolated and, as required, the particular cassette component analyzed as to its sequence to ensure that the proper sequence has been obtained. Depending upon the nature of the manipulation, the desired sequence may be excised from the plasmid and introduced into a different vector or the plasmid may be restricted and the expression cassette component manipulated, as appropriate.

The manner of transformation of E. Coli with the various DNA constructs (plasmids and viruses) for cloning is not critical to this invention. Conjugation, transduction, transfection or transformation, for example, calcium phosphate mediated transformation, may be employed.

In addition to the expression cassette, depending upon the manner of introduction of the expression cassette into the plant cell, other DNA sequences may be required. For example when using the Ti- or Ri-plasmid for transformation of plant cells, as described below, at least the right border and frequently both the right and left borders of the T-DNA of the Ti- or Ri-plasmids will be joined as flanking regions to the expression cassette. The use of T-DNA for transformation of plant cells has received extensive study and is amply described in Genetic Engineering, Principles and Methods (1984) Vol 6 (Eds. Setlow and Hollaender) pp. 253-278 (Plenum, NY) ; A. Hoekema, in : The Binary Plant Vector System (1985) Offsetdrukkerij Kanters, B.V. Alblasserdam.

Alternatively, to enhance integration into the plant genome, terminal repeats of transposons may be used as borders in conjunction with a transposase. In this situation, expression of the transposase should be inducible, so that once the expression cassette is integrated into the genome, it should be relatively stably integrated and avoid hopping.

The expression cassette will normally be joined to a marker for selection in plant cells. Conveniently, the marker may be resistance to a biocide, particularly an antibiotic, such as Kanamycin, G418, Bleomycin, Hygromycin, Chloramphenicol, or the like. The particular marker employed will be one which will allow for selection of transformed plant cells as compared to plant cells lacking the DNA which has been introduced.

A variety of techniques are available for the introduction of DNA into a plant cell host. These techniques include transformation with Ti-DNA employing A. tumefaciens or A. rhizogenes as the transforming agent, protoplast fusion,

injection, electroporation, DNA particle bombardment, and the like. For transformation with agrobacterium, plasmids can be prepared in E. coli which plasmids contain DNA homologous with the Ti-plasmid, particularly T-DNA. The plasmid may be capable of replication in Agrobacterium, by inclusion of a broad spectrum prokaryotic replication system, for example RK290, if it is desired to retain the expression cassette on a independent plasmid rather than having it integrated into the Ti-plasmid. By means of a helper plasmid, the expression cassette may be transferred to the A. tumefaciens and the resulting transformed organism used for transforming plant cells.

Conveniently, explants may be cultivated with the A. tumefaciens or A. rhizogenes to allow for transfer of the expression cassette to the plant cells, the plant cells dispersed in an appropriate selection medium. The Agrobacterium host will contain a plasmid having the vir genes necessary for transfer.

After transformation, the cell tissue (for example protoplasts, explants or cotyledons) is transferred to a regeneration medium, such as Murashige-Skoog (MS) medium for plant tissue and cell culture, for formation of a callus. Cells which have been transformed may be grown into plants in accordance with conventional ways. See, for example, McCormick et al., Plant Cell Reports (1986) 5 : 81-84. The transformed plants may then be analyzed to determine whether the desired gene product is still being produced in all or a portion of the plant cells. After expression of the desired product has been demonstrated in the plant, the plant can be grown, and either pollinated with the same transformed strain or different strains and the resulting hybrid having the desired phenotypic characteristic identified. Two or more generations may be grown to ensure that the subject phenotypic characteristic is stably maintained and inherited.

## 1 - **PURIFICATION OF SUCROSE PHOSPHATE SYNTHASE OF CORN**

### 1.1 - Method of determination of enzymatic activity (SPS)

During purification SPS activity is followed in 2 ways :

**a)** either by means of a colorimetric test (P.S. Kerr et al., Planta., 1987, 170 : 515-519) called resorcinol test described below.

Sucrose Phosphate Synthase or UDP glucose - D Fructose - Phosphate Glucosyltransferase (EC 2.4.1.14) catalyzes the reaction :

UDPG + Fructose 6-P <=> Sucrose 6-P + UDP

| | |
|---|---|
| UDPG : | Uridine Di-Phospho Glucose |
| Fructose 6-P or F6P : | Fructose 6-Phosphate |
| Sucrose 6-P : | Sucrose 6-Phosphate |

The sucrose 6-P formed reacts with the Resorcinol to give a red-colored compound quantifiable by spectrophotometry at 520 nm (nanometer) (Optical Density (O.D.) = 520 nm). In practice, to 45 µl (microliter) of enzymatic preparation 25 µl of a buffered solution containing the two substrates is added (UDPG 70 mM, F6P 28 mM, $MgCl_2$ 15 mM, HEPES 25 mM pH 7,5). After incubation at 37°C, reaction is stopped by adding 70 µl of NaOH in solution and heating at 95°C during 10 mn. After cooling, 0,25 ml of a solution 0,1 % resorcinol in ethanol 95 % is added, then 0,75 ml of HCl 30 % is added. The OD 520 mM is read after incubation 8 mn at 80 mn, and cooling.

**b)** or by means of a coupled enzymatic system (S. Harbron et al., Anal. Biochem. 1980, 107 : 56-59) being composed in the following way :

UDPG + F6P <=> Sucrose 6 P + UDP
SPS

UDP + ATP <=> ADP + UTP
Nucleoside Diphosphokinase $NP_2K$

ADP + PEP <=> Pyruvate + ATP
Pyruvate kinase PK

Pyruvate + NADH <=> NAD + lactate
Lactate dehydrogenase LDH

The disappearance of the NADH absorption at 340 nm is monitored 1 mole of NAD formed or 1 mole of NADH consumed corresponds to 1 mole of sucrose 6 P formed.

In pratice, in a quartz spectrophotometric tun thermostated at 37°C, the following solution are added.

- 540 µl of HEPES buffered 50 mM, $MgCl_2$ 10 mM KCl 20 mM pH=7.5,
- 250 µl of a mixture of substrates PEP (1,6 mM NaDH 0,6 mM, ATP 4 mM UDPG 112 mM),
- 60 µl of an enzyme mixture (LDH 166,7 U/ml PK 333,3 U/ml, NPzK 66,7 U/ml),
- 100 µl of F6P 112 mM.

After homogenization, 50 µl of the preparation containing SPS is added, the diminution of optical density at 340 nm is added with a spectrophotometer (UVIKON 860, KONTRON instruments). The measure is done with the kinetic of the machine.

1.2 - <u>Purification of the SPS (preparation of the immunogen)</u>

1.2.1 - **Extraction**

The starting material for the purification are mature leaves of young corn plants (<u>Zea mays</u> L. cv Pioneer 3184), which have been harvested in late morning, cut up, deveined, frozen in liquid nitrogen and stored at -70°C.

250 g of leaves are suspended in 1 liter of 50 mM HEPES 10 mM $MgCl_2$ 1 mM EDTA 5 mM DTT, pH=7.5 buffer (extraction buffer) which has observed to it 11 g of Polyvinyl-pyrrolidone, nitrogen is bubbled through and the suspension is cooled to 0°C.

The leaves are ground, until a homogeneous liquid is obtained. This ground product is filtered, and then centrifuged at 14,000 g for 20 minutes at 4°C.

While the bubbling through of nitrogen is maintained, a solution of 50 % Poly Ethylene Glycol (PEG 8000 "Breox" at 50 % w/v of extraction buffer) is added to the supernatant until a final concentration of PEG of 6 % is reached. Then the suspension is cooled at 0°C. After centrifuging at 14,000 g for 20 minutes the supernatant has added to it 50 % PEG until a final concentration of PEG of 12 % is reached. After a repeated centrifugation, the supernatant is discarded and the residue is solubilized with 60 ml of 50 mM HEPES, 10 mM $MgCl_2$, 1 mM EDTA, 5 mM DTT, 10 % Ethylene Glycol (EG), 0.08 M KCl, pH 7.5 buffer (recovery buffer). This solution is clarified by centrifuging at 40,000 g for 10 minutes. The supernatant constitutes the final extract.

1.2.2 - **Low pressure anion-exchange chromatography : fast-flow DEAE Sepharose exchanger**

The final extract is chromatographed on a column 25 mm x 162 mm of 80 ml of Fast-Flow DEAE Sepharose PHARMACIA equilibrated with recovery buffer. After washing the column with the same buffer, the proteins adsorbed on the support are eluted by means of a linear gradient with increasing ionic strength between 0.08 M KCl and 0.35 M KCl in the 50 mM HEPES, 10 mM $MgCl_2$, 1 mM EDTA, 5 mM DTT, 10 % EG, pH 7.5 buffer (buffer A). The flow rate applied during this experiment is 180 ml/h and chromatography is executed at 4°C.

The SPS activity is eluted at about 0.17 M KCl.

1.2.3 - **Chromatography on heparin Sepharose**

The fractions containing the SPS activity are collected and diluted to one fifth in buffer A, then added to 12 ml of heparin Sepharose previously equilibrated with buffer A. After one hour of incubation with gentle agitation at 4°C, the gel is washed with about 10 volumes of buffer A + 0.05 M KCl, then repacked in a chromatography column.

The proteins adsorbed are eluted in an isocratic way by means of a 10 mM CAPS, 10 mM $MgCl_2$, 1 mM EDTA, 5 mM DTT, 10 % EG, 0.01 % Tween 80, 1 mg/ml heparin, 1 % Fructose, 0.25 M KCl, pH 10 buffer, delivered at 60 ml/h.

Chromatography is executed at 4°C.

The fractions containing the SPS activity are collected (heparin fraction) and preserved on ice until the following purification stage. The enzyme at this stage is stable for a least one week.

The following purification steps are carried out using a system of High Performance Liquid Chromatography (HPLC) ; the purification is followed by means of a detector fitted with a filter enabling absorbency in the ultra-violet at 280 nm (A280) to be measured. The buffers and the fractions recovered are kept at low temperature.

1.2.4 - **High performance anion-exchange chromatography : Mono Q**

The heparin fraction is diluted by adding one third volume of 20 mM Triethanolamine, 10 mM $MgCl_2$, 1 mM EDTA, 10 mM DTT, 3 % EG, 0.3 % Tween 80, pH 7.5 buffer (buffer A) and loaded on an FPLC Mono Q HR10/10 column, (10 x 100 mm PHARMACIA) previously equilibrated with the same buffer which has added to it NaCl (final concentration 0.18 M). After the A280 has returned to 0, the proteins adsorbed on the chromatography support are eluted by means of a salt-complex gradient comprised as follows :

buffer A : cf above
buffer B : buffer A + NaCl 1 M

| time (minutes) | % B |
|---|---|
| 0 | 18 |
| 0.1 | 24 |
| 15 | 24 |
| 19 | 26 |
| 23 | 26 |
| 33 | 31 |
| 38 | 31 |
| 41 | 100 |
| 43 | 18 |

The flow rate applied to the column is 180 ml/h.

The SPS activity is eluted between 0.26 and 0.31 M NaCl.

The active fractions are collected together ("Mono Q fraction").

1.2.5 - **HPLC on Hydroxyapatite**

The Mono Q fraction is loaded on an HPLC column of hydroxyapatite 4 mm x 75 mm neutralized with 20 mM $KH_2PO_4/K_2HPO_4$, 3 % EG, 0.3 % Tween 80, 5 mM DTT, pH 7.5 buffer. After the A280 has returned to 0, the proteins adsorbed are eluted by means of the following phosphate gradient :

buffer A : cf above
buffer B : idem buffer A but 500 mM Phosphate of K

| time (minutes) | % B |
|---|---|
| 0 | 2 |
| 5 | 11 |
| 9 | 13 |
| 14 | 13 |
| 29 | 40 |
| 31 | 100 |
| 32 | 100 |
| 35 | 2 |

The flow rate applied is 60 ml/h. At this stage, the phosphate will partially inhibit SPS activity and therefore it is difficult to calculate a specific activity and also a purification factor (cf table 1) at this stage.

The SPS activity is eluted under these conditions with about 60 mM phosphate.

The active fractions are collected together and constitute the HAC fraction.

1.2.6 - **HPLC on DEAE 5PW**

The HAC fraction is loaded on an anion-exchange HPLC column of Di Ethyl Amino Ethyl type (DEAE-5PW) previously neutralized with a buffer of 20 mM Triethanolamine, 10 mM $MgCl_2$, 1 mM EDTA, 3 % EG, 2.5 mM DTT, 2 % betaine, pH 7.5 buffer (buffer A) + 0.15 M NaCl.

After the A280 has returned to 0, the proteins adsorbed are eluted by means of the following NaCl gradient :

buffer A : cf above
buffer B : idem buffer A with 1 M NaCl

| time (minutes) | % B |
|---|---|
| 0 | 15 |
| 0.1 | 20 |
| 5 | 20 |
| 22 | 35 |
| 27 | 35 |
| 30 | 100 |
| 31 | 15 |

The flow rate used is 60 ml/h.

The SPS activity is eluted with about 0.3M NaCl.

1.2.7 - **Preparation of the final preparation : concentration**

The final preparation is concentrated by HPLC chromatography on Mono Q HR5/5 exchanger (5 X 50 mm, Pharmacia) and rapid elution.

The DEAE 5PW fraction (or the G200 fraction) is diluted to two thirds with buffer A (idem 6) and loaded on the column previously neutralized with buffer A + 0.18 M NaCl. The following gradient is then applied on the column :

buffers A and B : idem 6

| time (minutes) | % B |
|---|---|
| 0 | 18 |
| 10 | 40 |
| 12 | 100 |
| 13 | 18 |

The flow rate used is 60 ml/h.

The SPS activity is eluted with about 0.3 M NaCl.

The final preparation is stored at -20°C until used. Table 1 summarizes the results obtained at the various purification stages in terms of quantities of proteins recovered and of SPS activity.

TABLE 1

| | Concentration of proteins (mg/ml) | Volume (ml) | sA** (U) | pF** | Y* (%) |
|---|---|---|---|---|---|
| Ground product | 1 | 1000 | 0.05 | 0 | 100 |

Observations : ** the measurement of the quantity of proteins is carried out using the Bradford method. As Tween interferes enormously with this method, it is not possible to determine the proteins and then to calculate an sA at the level of the stages containing one. Furthermore, as phosphate is an inhibitor of SPS activity, the determination during the HAC stage gives an underestimated result.

* the increasing yield during the initial stages can be explained by the elimination, during purification, of certain inhibitors of SPS activity.

TABLE 1 (continued)

| | Concentration of proteins (mg/ml) | Volume (ml) | sA** (U) | pF** | Y* (%) |
|---|---|---|---|---|---|
| Final extract | 4< <8 | 60 | 0.30 | 6 | 144 |
| DEAE FF fraction | 0.4< <0.8 | 70 | 3 | 60 | 168 |
| Heparin fraction | 0.2< <0.4 | 25 | 9 | 180 | 90 |
| Mono Q fraction | (0.02) | 30 | - | - | - |
| HAC fraction | (0.03) | 8 | - | - | - |
| Final preparation | 0.05 | 2 | 25 | 500 | 5 |

Key
sA = Specific enzymatic activity : 1 U corresponds to 1 µm of sucrose formed per minute per mg of protein at 37°C
pF = Purification factor
Y = Yield
( ) = approximate value
- = not determined

Observations : ** the measurement of the quantity of proteins is carried out using the Bradford method. As Tween interferes enormously with this method, it is not possible to determine the proteins and then to calculate an sA at the level of the stages containing one. Furthermore, as phosphate is an inhibitor of SPS activity, the determination during the HAC stage gives an underestimated result.

* the increasing yield during the initial stages can be explained by the elimination, during purification, of certain inhibitors of SPS activity.

An SDS-PAGE profile at various stages of the purification process and the quality of the final preparation is given in Figure 1. The 120, 95 and 35 kd proteins are correlated to the SPS activity.

The 35 and 95 kd proteins are very likely breakdown products of the 120 kd protein as it can be shown by the nucleotidic sequence coding for the SPS protein.

Furthermore, the antibodies directed against the 35 and 95 kd proteins also recognize the protein 120 kd in immuno-detection after membrane transfer, which demonstrates an antigenic identity between these three proteins (see below). It must be pointed out, however, that the addition of protease inhibitors in the buffers during purification has not enabled us to obtain a single 120 kd protein.

Gel permeation chromatographies were carried out in order to find the apparent molecular weight of the native SPS protein. Briefly, the HAC fraction is concentrated by HPLC chromatography on Mono Q HR 5/5 inchanger (see 1-2-7). The active fractions are collected together (about 2 ml) and loaded on an G 200 column previously washed with a buffer containing 20 mM triethanolamin, 10 mM $MgCl_2$, 1 mM EDTA, 3 % E.G., 2,5 mM DTT, 2 % betain, 0,3 M NaCl pH 7,5. The SPS activity is eluted a major protein peak corresponding to an apparent mass of 270-280 kda which is in agreement with the results obtained by S. HARBRON an al. (Arch. Biochem. Biophys., 1981, 212 : 237-246) with the spinach SPS. It can be noted that the chromatography on a TS lambda 60000 permeation column lead to the elution of the SPS activity at a retention time corresponding to an apparent mass of 440 kda which is near from the value obtained by DC. DOEHLERT and S.C. HUBER (Plant Physiol., 1983, 73 : 989-994) with the spinach SPS, using a AcA34 permeation column.

The SPS protein seems therefore to be a di or tetrameric protein having as the basic sub-unit a 120 kda protein (homo-dimeric or homo-tetrameric).

Key for Figure 1

SDS-PAGE profile of sucrose phosphate synthase of corn : 8.5 % acrylamide gel, reducing conditions and staining with silver nitrate

M : Standard of molecular weight B-Galactosidase (116 kd), bovine Albumin (68 kd), Egg Albumin (45 kd), carbonic Anhydrase (29 kd).
H : Heparin fraction, 30 micrograms of proteins per well.
FP : Final Preparation, 7.5 micrograms of proteins per well.
FE : Final Extract, 7.5 micrograms of proteins per well.
D : Fast-Flow DEAE fraction, 7.5 micrograms of proteins per well.

The bands of proteins visible at about 120 kd (1), 95 kd (2) and 35 kd (3) are correlated, during the chromatography stages, with the appearance of SPS activity in the respective fractions.

## 2 - **PROCESS FOR THE PREPARATION OF MONOCLONAL ANTIBODIES DIRECTED AGAINST SUCROSE PHOSPHATE SYNTHASE**

### 2.1 - Immunisations

BALB/C mice are immunized by subcutaneous injection (pads and paws) according to the following methodology :

Day 0 injection of about 5 micrograms of proteins (or about 0.3 U SPS per mouse) : Mono Q pool emulsified volume for volume with Freund's Complete Adjuvant (FCA).

Day 14 injection of about 5 micrograms of proteins (or about 0.3 U SPS per mouse) : Mono Q pool emulsified volume for volume with Freund's Incomplete Adjuvant (FIA).

Day 27 Idem D14

Day 0 + 60 injection of about 20 micrograms of proteins : final pool in FIA

Day 0 + 90 injection of about 12 micrograms of proteins : final pool in FIA

Day 0 + 135 injection by intravenous route (IV) in the tail of about 20 micrograms of proteins : final pool. Fusion is achieved 3 days after the IV immunisation.

The sera are removed at D34, D61, D98 and D159 in order to measure the immunitary response (cf screening).

### 2.1.1 - **Screening method**

As the SPS used for the immunisations is not perfectly homogeneous, it is necessary to establish a screening test specific to this enzyme.
Two methods for the detection of the antibodies are used :

- detection method of antibodies inhibiting the SPS activity
- detection method of antibodies directed against the SPS (inhibiting or not).

**a)** Detection method of antibodies inhibiting the SPS activity This method of screening allows the detection of antibodies which interfer with the active site of the SPS or on a site close to the latter, and therefore prevent the access of substrates. In practice, 70 µl of serum or of supernatant of hybridoma culture diluted in a suitable way is mixed with 70 µl of SPS preparation (Heparin fraction). After one hour of incubation at ambient temperature, the residual SPS activity is determined by coupled enzymatic determination (Cf 1-1). The results are expressed as a percentage of inhibition as compared to the same SPS preparation treated in the same way but without antibodies.

b) Detection method of antibodies directed against SPS (inhibiting or not)

This method is based on the precipitation of the anti-body-SPS complex by goat anti-mouse IgG coupled to sepharose beads (GAM sepharose). In practice, 60 µl of serum or supernatant of hybridoma culture diluted in any suitable manner is added to 60 µl of SPS preparation (Heparin fraction). After 2 hours of incubation at ambient temperature, the mixture is added to 50 µl of 25 % GAM-Sepharose previously washed three times with a buffer of 50 mM HEPES, 10 mM $MgCl_2$, 1 mM EDTA, 10 % EG, 5 mM DTT, pH 7.5. The mixture is incubated over night at 4°C under strong agitation. After centrifuging for 5 minutes at 3000 rpm the residual SPS activity in the supernatant is determined by coupled enzymatic determination (Cf 1.1). The results are expressed as a percentage of precipitation (% prec.) compared to the same SPS preparation treated in the same way without antibodies.

### 2.1.2 - **Results**

10 mice were immunized according to the protocol described previously. The following table gives the results of the precipitation determinations carried out with the heteroantisera of the 10 mice on D159. The sera are diluted to one two-hundredth.

| MOUSE | 1 | 2 | 3 | 4 | 5 | 6 | 7 | 8 | 9 | 10 |
|---|---|---|---|---|---|---|---|---|---|---|
| % PREC. | 45 | 22 | 32 | 64 | 36 | 30 | 22 | 16 | 39 | 37 |

Additional dilutions of the serum of mouse 4 give the following results :

| DILUTION | % PRECIPITATION |
|---|---|
| 1/200 | 67 |
| 1/400 | 48 |
| 1/600 | 29 |
| 1/1000 | 20 |

The spleens of mice 1 and 4 are used for the fusion.

2.2 - **Cellular fusion**

The splenocytes of the mice are fused with myeloma cells of SP2/0-Ag14 mice according to a ratio of 2/1 in the presence of 45 % polyethylene glycol 1500. The selection of the hybridomas is effected by adding hypoxanthine and azaserine to the culture medium 24 and 48 hours after fusion.

The hybridomas are cloned and sub-cloned by the method of limited dilution.

2.2.1 - **Results of the screening of hybrids and clones.**

| HYBRIDS | |
|---|---|
| MOUSE 4 (SPA fusion) 2 postive hybrids out of 45 | MOUSE 1 (SPB fusion 6 positive hybrids out of 52 |
| SPA2 : 38 % prec.<br>SPA19 : 7 % prec. | SPB3 : 17 % prec.<br>SPB5 : 67 % prec.<br>SPB8 : 53 % prec.<br>SPB13 : 68 % prec.<br>SPB25 : 13 % prec.<br>SPB34 : 17 % prec. |

| CLONES | |
|---|---|
| SPA FUSION 2 clones retained out of 36 | SPB FUSION 7 clones retained out of 46 |
| SPA2-2 : 85 % prec.<br>SPA19-7 : 8 % prec. | SPB3-2 : 19 % prec.<br>SPB5-1 : 76 % prec.<br>SPB5-2 : 71 % prec.<br>SPB5-3 : 45 % prec.<br>SPB5-4 : 24 % prec.<br>SPB13-1 : 79 % prec.<br>SPB13-2 : 53 % prec. |

| SUB-CLONES | |
|---|---|
| SPA FUSION 3sub-clones retained out of 48 | SPB FUSION 5 sub-clones retained out of 72 |
| SPA2-2-3 : 60 % prec.<br>SPA2-2-22 : 33 % prec.<br>SPA2-2-25 : 92 % prec. | SPB3-2-19 : 21 % prec.<br>SPB5-2-10 : 86 % prec.<br>SPB5-4-2 : 46 % prec.<br>SPB13-1-7 : 87 % prec. |

(continued)

| SUB-CLONES | |
|---|---|
| SPA FUSION 3sub-clones retained out of 48 | SPB FUSION 5 sub-clones retained out of 72 |
| | SPB13-2-2 : 93 % prec. |

2.2.2 - **Production of anti-SPS monoclonal antibodies**

The hydridomas are injected by intra-peritoneal route into female BALB/C mice previously treated with pristane. The monoclonal antibodies are partially purified from ascital liquids thus produced by precipitation with 18 % sodium sulphate. The proteins precipitated are dissolved then dialyzed against PBS (F18).

2.2.3 - **Characterization of anti-SPS monoclonal antibodies**

**a)** Typing

The typing is done using an ELISA test. Anti-IgG rabbit and anti-IgM mouse antibodies (ZYMED) are fixed at the bottom of the wells of a 96-well plate. After one night at ambient temperature the unoccupied sites are saturated with a solution of 3 % bovine serum albumin in PBS. After one hour of incubation at 37°C and several washes, the various F18's are deposited in the wells. After incubation and several washes, goat or rabbit antibodies, anti-class and anti-sub class mouse immunoglobulins linked with peroxidase, are added. After one hour at 37°C, the antibodies are revealed using an $H_2O_2$/ABTS system.

All the anti-SPS monoclonal antibodies were found to be of Ig $G_1$ type.

**b)** Inhibition of SPS activity

The determination of the capacity of the antibodies to inhibit the SPS activity is carried out by the technique mentioned previously (Cf 2.1.1 a) using F18's.

| | Concentration of antibodies (micrograms/ml) | % Inhibition |
|---|---|---|
| Antibody SPA2-2-3 | 50 | 0 |
| SPA2-2-22 | 50 | 0 |
| SPA2-2-25 | 50 | 0 |
| SPB3-2-19 | 50 | 0 |
| SPB5-2-10 | 50 | 0 |
| SPB5-4-2 | 50 | 0 |
| SPB13-1-7 | 50 | 50 |
| | 25 | 55 |
| | 5 | 25 |
| | 2.5 | 10 |
| | 1 | 2.1 |
| SPB13-2-2 | 50 | 60.1 |
| | 25 | 59.1 |
| | 5 | 33.8 |
| | 2.5 | 14.2 |
| | 1 | 8.7 |

**c)** Immuno-precipitation of the SPS activity

The determination of the capacity of the antibodies to immuno-precipitate the SPS activity is carried out by the technique mentioned previously (Cf 2.1.1 b) using F18's.

| | Concentration of antibodies (micrograms/ml) | % Precipitation |
|---|---|---|
| Antibody SPA2-2-3 | 50 | 95 |
| | 25 | 92 |
| | 5 | 80 |
| | 2.5 | 40 |

(continued)

| | Concentration of antibodies (micrograms/ml) | % Precipitation |
|---|---|---|
| | 1 | 20 |
| SPA2-2-22 | 50 | 95.7 |
| | 25 | 95 |
| | 10 | 51 |
| | 5 | 48.2 |
| | 2.5 | 25 |
| | 1 | 10 |
| SPA2-2-25 | 50 | 91.3 |
| | 25 | 95.3 |
| | 5 | 90.4 |
| | 2.5 | 22.8 |
| | 1 | 12.5 |
| SPB3-2-19 | 50 | 95 |
| | 25 | 95 |
| | 5 | 27.8 |
| | 2.5 | 17.8 |
| | 1 | 9.3 |
| SPB5-2-10 | 50 | 95 |
| | 25 | 95 |
| | 5 | 81.1 |
| | 2.5 | 41.4 |
| | 1 | 22.6 |
| SPB5-4-2 | 50 | 95 |
| | 25 | 95 |
| | 5 | 86.1 |
| | 2.5 | 57.2 |
| | 1 | 26.1 |
| SPB13-1-7 | 50 | 95 |
| | 25 | 95 |
| | 10 | 65.4 |
| | 5 | 48.1 |
| | 2.5 | 15 |
| | 1 | 10 |
| SPB13-2-2 | 50 | 95 |
| | 25 | 95 |
| | 5 | 71.8 |
| | 2.5 | 43.5 |

3 - **USE OF THE MONOCLONAL ANTIBODIES FOR THE CHARACTERIZATION AND PURIFICATION OF SUCROSE PHOSPHATE SYNTHASE**

3.1 - Characterization of Corn Sucrose Phosphate Synthase

This characterization is carried out with SPB3-2-19 and SPB13-2-2 antibodies by the technique of immuno-detection after transfer of the proteins from an electrophoresis gel under denaturing conditions (SDS-PAGE) on nitrocellulose membrane (western).

After electrophoretic separation in a 12.5 % acrylamide gel (Nature 277 (1970) 680-685), the proteins are transferred onto a 0.22 µm nitrocellulose membrane (Schleicher and Schuell). The buffer is a standard electrophoresis buffer (3.03 g/l. TRIS base, 14.4 g/l. Glycine, 0.1 % SDS, pH 8.3, 20 % methanol).

After transfer, the membrane is put in a blocking bath (0.5 % Casein in PBS). After one hour at 37°C under gentle

agitation, the membrane is washed 3 to 4 times in a washing buffer (0.1 % Casein, 0.5 % Tween 20, in PBS) then incubated with a solution of 10 micrograms/ml of the monoclonal antibody to be tested. A part of the membrane is incubated in parallel with a non-immune anti-body (negative control). After one hour of incubation at ambient temperature followed by 9 or 10 washes, the membrane is incubated in the presence of an anti-mouse antibody antibody labelled with Iodine 125 diluted in a washing buffer (50,000 cpm per $cm^2$ of membrane). After one hour of incubation at ambient temperature followed by 9 or 10 washes, the membrane is dried, then autoradiographed (X-OMAT AR KODAK film and Cronex XTRA Life DUPONT amplifying screen). An autoradiography is shown in Figure 2. A strong signal is observed at the protein bands 120 kd, 95 kd and 35 kd which correlates with the previous results (see first part).

Key to Figure 2

A : membrane incubated in the presence of the SPB3-2-19 antibody
B : membrane incubated in the presence of an antibody not directed against SPS (negative control anti-neomycin monoclonal antibody)
C : membrane incubated in the presence of the SPB13-2-2 antibody
M : standards of molecular weight radio-marked by $125_I$ (NEX-188 NEN) B-Galactosidase (116 kd), bovine albumin (68 kd), carbonic Anhydrase (29 kd), trypsic Inhibitor (20.1 kd), Alpha-Lactalbumin (14.4 kd), 150,000 cpm per lane
PA : proteins obtained after immunoaffinity chromatography (see below) with the SPB13-2-2 monoclonal antibody, about 40 micrograms of proteins per lane.
H : Heparin fraction, about 40 micrograms of proteins per lane.

3.2 - Purification of Sucrose Phosphate Synthase by immunoaffinity Chromatography

A methodology for the purification of corn Sucrose Phosphate Synthase on an immunoaffinity support has been perfected in order to increase the quantity of protein recovered while reducing the number of purification stages and to obtain quantities sufficient for protein sequencing.

3.2.1 - **Preparation of the immuno-adsorbent**

The F18 (see 2.2.2) corresponding to the SPB13-1-7 anti-body or to the SPB13-2-2 antibody were mixed with activated CH-Sepharose, (1 mg of antibody per ml of gel). After incubation for 2 hours at ambient temperature, the sites not occupied by the antibodies are saturated with 1M ethanolamine, pH 9. The support is then washed alternately with 0.1M acetate 0.5 M NaCl pH 4 buffer and 0.1 M TRIS 0.5 M NaCl pH 8 buffer. The immunoaffinity support thus prepared is preserved at 4°C in a 50 mM HEPES, 10 mM $MgCl_2$, 1 mM EDTA, lmM PMSF, 0.01 % sodium nitride (azide), pH 7.5 buffer.

3.2.2 - **Immunoaffinity Chromatography**

50 % PEG is added to the Heparin fraction of SPS (see 1.2.3.) is added 50 % PEG (see 1.2.1) to a final concentration of PEG of 20 %. After incubation for 30 minutes at 4°C with gentle agitation, the mixture is centrifuged at 1600 g for 30 minutes. The protein deposit is taken up in half of the initial volume with the 50 mM HEPES, 10 mM $MgCl_2$, 1 mM EDTA, 10 % ethylene glycol pH 7.5 buffer. This stage allows the previous buffer, which is incompatible with the immunoaffinity chromatography, to be eliminated, and the proteins to be concentrated. The yield of SPS activity is from 80 to 90 %.

The solution obtained is applied with a flow rate of 0.1 ml/min over 1 ml of immunoaffinity support packed in a column and on which has been fixed an antibody not directed against the SPS (activated CNBr-Sepharose, on which an anti-neomycin antibody is fixed). This first stage allows the elimination of certain contaminants which are fixed non-specifically on the chromatography support. The effluent of the non-specific column is in its turn applied to the anti-SPS immunoaffinity support (2 ml in an 11 x 20 mm column) with a flow rate of 0.1 ml/min. These two stages are carried out at laboratory temperature. The column is washed with 10 ml of load buffer and then with a washing buffer (load buffer with the addition of 0.25 M NaCl and 0.3 % Tween 20) until absorbency in ultra-violet at 280 nm is close to base level. The proteins adsorbed on the support are eluted with a solution of 50 mM triethyl-amine, pH 11. This elution is carried out at 4°C and the immunoaffinity column is reversed to obtain an optimum yield. The SDS-PAGE profile of the final preparation obtained corresponds to that obtained using the standard protocol (see 1). It must be noted that the elution method of the proteins adsorbed on the immunoaffinity support irrevesibly destroys the SPS activity but the recovery yield of the eluted SPS proteins is optimal compared to tests carried out in native elution conditions. The eluate of the immunoaffinity column is desalted with a Sephadex G25 column, against a 0.14 % Glycerol, 0.07 % 2-mercapto-ethanol, 0.04 % SDS, 0.9 mM TRIS pH 6.8 buffer (electrophoresis buffer in reducing conditions diluted 70

times). After desalification, the protein preparation is concentrated 70 times with a concentrator under vacuum and the SPS proteins are purified by SDS-PAGE (see below).

4. Partial Sequencing of SPS Polypeptides

Samples of a purified protein preparation obtained as described in Example 3.2.2. were subjected to preparative SDS-PAGE. After electrophoresis, the protein bands were visualized with KCl treatment as described by Bergman and Joernvall (Eur. Jour. Biochem. (1978) 169 : 9-12) and the bands observed at 90kd and 30kd were excised. The proteins from these gel fragments were electroeluted using an Electrophoretic Concentrator according to manufacturer's instructions (ISCO ; Lincoln, NE) in 4 mM sodium acetate, pH8. After electroelution, protein yields were quantitated by comparison to a bovine serum albumin (BSA) standard on a Coomassie Blue-stained gel. Approximately 30 µg of the 30 kd protein and 75 µg of the 90 kd protein were obtained.

The proteins were concentrated by acetone precipitation, and resuspended in 50 mM ammonium carbonate buffer, pH8. Tryptic digestion and HPLC purification were performed as described by Sturm and Chrispeels (Jour. Biol. Chem. (1987) 262 : 13392-13403). Briefly, digestion was performed by addition of trypsin (5 % of SPS protein), and incubation for two hours at 37°C. The digestion was then repeated. The proteins were concentrated by lyophilization and resuspended in 50mM sodium phosphate buffer, pH2.2. This mixture was subjected to reverse phase HPLC separation by application to a C18 column in phosphate buffer. Elution was performed using an increasing gradient of acetonitrile. Eluted material from the phosphate buffer/acetonitrile gradient was monitored at 214 nm. The fractions corresponding to peaks of absorbance at 214 nm were collected, lyophilized, resuspended in 0.1 % trifluoroacetic acid, reapplied to the C18 column (equilibrated with 0.1 % trifluoroacetic acid), and eluted using an acetonitrile gradient. Eluted material from the trifluoroacetic acid/acetonitrile gradient was monitored at 214 nm. The fractions corresponding to peaks of absorbance at 214 nm were collected, lyophilized, and subjected to standard Edman degradation protein sequencing on an automated protein sequencer (Applied Biosystems ; Foster City, CA). Sequences of 5 peptides were obtained. **Fig. 3.**

5. Isolation and Assembly of a Full-Length cDNA for SPS

5.1. RNA Isolation from Corn Leaf

Total RNA was isolated from corn leaves (see 1.2.1.) according to the method of Turpen and Griffith (Biotechniques (1986) 4 : 11-15). Briefly, 250 gm of material was homogenized in 4M guanidine thiocyanate and 2 % sarcosyl. The mixture was then centrifuged and the cleared supernatant was layered upon a 5.7M CsCl cushion and centrifuged for 5.5 hours at 50,000 RPM. The RNA pellet was dissolved in water, extracted with phenol and chloroform, and precipitated with ethanol. The resulting pellet was resuspended in water. The final yield of the RNA isolation was quantitated by UV spectrophotometry.

5.2. Poly(A) RNA Isolation

A saturated suspension of cellulose powder/water was added to the RNA/water mixture, at 10 % of the total volume, to remove residual polysaccharides. After centrifugation, the supernatant, containing the RNA, was applied to an oligo (dT)-cellulose column as described by Maniatis et al. (Molecular Cloning : A Laboratory Manual, (1982) Cold Spring Harbor, New York). The fraction containing the poly(A)+ RNA was then reapplied to the column. The eluted fraction containing the poly(A)+ RNA was extracted with phenol, and the RNA was precipitated with ethanol. Analysis by gel electrophoresis showed complete absence of ribosomal RNA.

5.3. Construction of Total Corn Leaf Library

cDNA synthesis was performed according to manufacturer's instructions (RiboClone cDNA Synthesis System by Promega, Madison, WI), using five µg of poly(A)+ RNA as template, except that M-MLV reverse transcriptase (BRL ; Bethesda, MD) was substituted for AMV reverse transcriptase. EcoRI linkers were added to the blunt-ended cDNA, and the resulting fragments were cloned into an expression vector (LambdaZAP, Stratagene ; La Jolla, CA) according to manufacturer's instructions. The resulting library contained approximately 1.5 x $10^6$ transformants.

5.4. PCR Generation of a Partial SPS cDNA Probe

Using the sequence information from the peptides of Example 4 and the polymerase chain reaction (PCR), a 1200 bp SPS cDNA fragment was generated. Total corn leaf cDNA (5.3.) was used as a template, and degenerate oligonucleotides, designed from two peptide sequences of the 30kd and 90kd SPS polypeptides, were used as primers. These primer sets were designated as CD3 and CD4. **Fig. 4**. PCR was carried out, according to manufacturer's instructions (GeneAmp DNA Amplification Reagent Kit and DNA Thermal Cycler of Perkin Elmer Cetus ; Norwalk, CT) except that the reaction was carried out for 30 cycles, and the annealing steps were programmed to be 50°C for 1 minute. The PCR reactions were analyzed by agarose gel electrophoresis. Use of the correct set of primers, which was CD3, resulted in a 1200 bp band being generated by the PCR reaction. PCR using the other set of primers, CD4, gave no specific signals. **Fig. 5**. Southern analysis confirmed that the PCR band was not an artifact, as shown in **Fig. 5**. The probe 4K5 was used in that the corresponding sequence of the probe was predicted to be within the 1200bp fragment if the fragment corresponded to the SPS sequence. The probe hybridized to the 1200 bp band generated by PCR using the primer set CD3 but not to PCR products generated by the primer set CD4 **Fig. 5**.

5.5 Isolation of SPS Bacteriophage Lambda cDNA Clones

The 1200 bp PCR-generated fragment was labeled with $^{32}P$ (as per the Random Primed DNA Labeling Kit, Boehringer Mannheim, Indianapolis, IN) and used as a probe to screen approximately 250,000 plaques of the cDNA library (5.3.). The inserts of the positive clones were analyzed by restriction analysis with EcoRI, and the clones with the longest inserts, SPS#3 and SPS#18, were selected for further analysis. **Fig. 6.** A 0.4 kb HindIII/EcoRI fragment from the 5' end of SPS#3 was isolated, then labeled with $^{32}P$ by random priming (Random Primed DNA Labeling Kit) and used as a probe to re-screen the library. Another clone, designated SPS#61, which extends further upstream than SPS#3, was isolated. **Fig. 6.** DNA sequencing indicated that the 5' end of the SPS reading frame was not reached.

To isolate cDNA clones that included more of the 5' region than SPS#3 or SPS#61, a new cDNA library was prepared, as per Example 5.3., (RiboClone cDNA Synthesis System by Promega ; Madison, WI) using M-MLV reverse transcriptase instead of AMV reverse transcriptase. However, instead of using oligo (dT) as a primer, a synthetic 17 bp primer, 23B, derived from the 5' sequence of the SPS#61 clone, was used (**Fig. 6**). This resulted in cDNAs that only contain regions upstream of the the SPS#61 5' region. The library was screened with the $^{32}P$-labeled EcoRI insert from SPS#61, and 16 positive clones were obtained. The clones with the longest inserts, SPS#77 and SPS#90, were selected for further analysis. DNA sequencing of SPS#77 and SPS#90 showed that the region of overlap (greater than 100 bp) with SPS#61 was identical in all clones, and that both extend further upstream into the 5' region. **Fig. 6**

PCR was carried out using single-stranded cDNA (from a reverse transcriptase reaction corn leaf RNA (5.2.) primed with oligo (dT) T) as described above) as template and primers selected from the SPS#90 and SPS#3 sequences, confirmed that SPS#90 and SPS#3 originate from the same mRNA transcript. The fragment resulting from this PCR reaction was 750 bp in length, consistent with the the size predicted from the DNA sequence. The 750 bp fragment was subcloned into a Bluescript-derived vector as a SalI/HindIII fragment. Four of the resulting subclones were partially sequenced, and the sequence obtained matched the existing DNA sequence.

5.6. Assembly of the SPS Reading Frame.

Both DNA strands of #90, #61, and #3 were sequenced, using the method of Sanger et al. (PNAS (1977) 74 : 5463-5467). All three sequences can be combined to one contiguous sequence of 3509 bp, **Fig. 7**. Primer extension experiments using corn leaf poly(A) RNA and an antisense primer showed that the 5' end of our DNA sequence represents sequences form the actual 5' end of the SPS in RNA. In the SPS reading frame, as defined by the five peptide sequences (**Fig. 3**), the first methionine codons are located at bp 112 and bp 250. **Fig. 7**. The codon at bp 112 is similar to the consensus eukaryotic translational start site (Kozak, Cell (1986) 44 : 283-292) and is located 54 bp downstream of a TAG stop codon (bp 58). It is proposed that this codon represents the translational start of the SPS polypeptide in vivo. After a 1068 codon reading frame, translation is stopped by TGA. The following 193 bp contain the 3' untranslated region including a poly(A) addition signal, AAATAAA.

The full-length SPS coding region may be assembled by combining the 529 bp BamHI/HindIII fragment of SPS#90, the 705 bp HindIII fragment of SPS#61 and the 2162 bp HindIII/ EcoRI fragment from SPS#3 (see Fig. 6).

## 6. **Detection of SPS Polypeptides by Specific Antisera**

Samples of purified protein preparations obtained by the method described in 3.2.2. were subjected to SDS-PAGE electrophoresis. The proteins in the gel were fixed and stained. The bands corresponding to the 90kd and 30kd polypeptides were excised. With this material polyclonal antisera were raised in rabbits by conventional procedures. Western analysis (as described by Oberfelder, Focus (1989) 11(1) : 1-5) showed that the antibodies isolated from the rabbit immunized with SPS 30 recognized the bands corresponding to the SPS 30 and SPS 120 peptides on a SDS PAGE gel, and that the antibodies isolated from the rabbit immunized with SPS 90 recognized the bands corresponding to the SPS 90 and SPS 120 polypeptides **Fig.8**.

6.2. Immunological Localization of SPS in the Corn Plant

Total proteins were extracted from leaves of a 30 day-old corn plant, harvested at 11 : 00 AM, by boiling in SDS buffer. The protein extracts were loaded on duplicate SDS-PAGE gels. One gel was stained with Coomassie Blue, while the other was subjected to Western analysis, using a mixture of SPS30 and SPS90 antisera as probe. **Fig. 9**. The prominent bands appearing on the Coomassie Blue-stained gel were identified as phosphoenolpyruvate carboxylase (PEPcase), an enzyme involved in C4 photosynthesis. The Western blot showed the presence of the SPS band. The SPS protein pattern was very similar to the PEPcase protein pattern : not present in roots, and not present in the section of leaf closest to the the stem, or in very young leaves. This pattern corresponds with expression associated with photosynthesis, and is the pattern expected for SPS.

## 7. **Construction of Expression Constructs Plasmids**

### 7.1. Construction of the full-length SPS reading frame

Clone SPS#90 is digested with HindIII and ligated with the 705 bp HindIII fragment from clone SPS#61 to create a plasmid containing the 5' end of the SPS coding region. The resulting plasmid is digested with BamHI and partially digested with HindIII, resulting in a 1340 bp BamHI/HindIII fragment containing the 5' end of the coding region. The 3' end of the SPS coding region is obtained by digestion of SPS#3 with EcoRI and partial digestion with HindIII, resulting in a 2162 bp HindIII/EcoRI fragment. This 2162 bp HindIII/EcoRI fragment, carrying the 3' end, is ligated with the 1340 BamHI/EcoRI fragment carrying the 5' end into a BamHI/EcoRI-digested pUC-derivative plasmid Bluescript, to create a plasmid carrying the entire 3403 bp SPS coding region and 3' untranslated transcription termination region.

### 7.2 Expression of SPS in E. coli

When cloning the 3403 bp BamHI/EcoRI SPS fragment into the plasmid Bluescript SK (Stratagene, La Jolla, CA), a translational fusion between the plasmid coded lacZ sequence and the SPS reading frame is created. The resulting fusion protein contains 30 N-terminal amino acids from the betagalactosidase and the complete SPS polypeptide. The fusion protein was expressed in E. coli under the Bluescribe plasmid lacZ promoter. Preparation of total protein followed by Western analysis using anti SPS antisera (6.1.) shows a band comigrating with native plant SPS. For SPS activity test the E. coli cells containing the SPS expression construct as described were opened with Lysozyme and sonication. Soluble protein was desalted by a Sephadex G-25 column. This protein extract was assayed for SPS activity analogous to (1.1.a.) except the reagent anthrone was used instead of resovcinol (E.U. Handel, Analytical Biochemistry, (1968) 22 : 280-283). This test shows that the SPS protein, expressed from the cDNA in E. coli does have SPS enzyme activity. By comparison to native plant enzyme it seems to have the same specific activity.

### 7.3. Construction of the Tobacco Small Subunit (SSU) Promoter-Transcriptional Fusions

The SPS coding region can be conveniently cloned as a BamHI/EcoRI (bp 106 - bp 3506) fragment 3' of a tobacco small subunit promoter.

A SSU promoter for expression of the SPS coding region, may be prepared as follows. The SSU promoter region from **PCGN627**(described below) is opened by KpnI and the 3' overhang removed. After EcoRI digestion, the 3403 bp BamHI (filled in) EcoRI SPS cDNA fragment (see, Example 7.1.) can be inserted.

After the SPS coding region is ligated into the SSU promoter, the SSU/SPS region may be ligated into a binary vector and integrated into a plant genome via Agrobacterium tumefaciens mediated transformation (The SPS region carries its own transcription termination region in the cDNA sequence.)

Insertion of the ssu/SPS construct into the binary vector pCGN1557 results in pCGN3812.

**pCGN627**

The 3.4 kb EcoRI fragment of TSSU3-8 (O'Neal et al., Nucleic Acids Res. (1987) 15 : 9661-8677), containing the small subunit promoter region, is cloned into the EcoRI site of **M13mp18** (Yanisch-Perron et al, Gene (1985) 53 : 103-119) to yield an M13 clone **8B**. Single-stranded DNA is used as a template to extend oligonucleotide primer "Probe 1" (O'Neal et al., Nucleic Acids Research (1987) 15 : 8661-8677) using the Klenow fragment of DNA polymerase I. Extension products are treated with mung bean nuclease and then digested with HindIII to yield a 1450 bp fragment containing the SSU promoter region. The fragment is cloned into HindIII-SmaI-digested pUC18 (Yanisch-Perron et al., Gene (1985) 53 : 103-119) to yield **pCGN625**.

**pCGN625** is digested with HindIII, the ends blunted with Klenow, and the digested plasmid re-digested with EcoRI.

The EcoRI/blunted-HindIII fragment containing the SSU promoter region is ligated with SmaI/EcoRI-digested **pUC18** to yield **pCGN627**.

7.4. Construction of a CaMV Promoter - SPS Transcriptional Fusion

The 35S promoter DNA fragment from cauliflower mosaic virus can be fused to the SPS DNA as follows.

The plasmid pCGN639 can be opened by BamHI and EcoRI and the 3403 bp BamHI/EcoRI SPS cDNA fragment as described in Example 7.1 can be cloned into this plasmid. The hybrid gene can be removed from this plasmid as a 4.35 kb XbaI EcoRI fragment and ligated into a binary vector (K.E. McBride and K.R. Summerfelt, Plant Mol. Bio. (1990) 14 : 269-276) and integrated into a plant genome via Agrobacterium tumefaciens mediated transformation. Insertion of the CaMV/SPS construct into the binary vector pCGN1557 (McBride and Summerfelt supra) results in pCGN3815.

7.4.1. Construction of pCGN639

**pCGN164** is digested with EcoRV and BamHI to release a EcoRV-BamHI fragment which contained a portion of the 35S promoter (bp 7340-7433). **pCGN638** is digested with HindIII and EcoRV to release a HindIII-EcoRV fragment containing a different portion of the 35S promoter (bp 6493-7340). These two fragments are ligated into **pCGN986** which has been digested with HindIII and BamHI to remove the HindIII-BamHI fragment containing the 35S-promoter ; this ligation produces **pCGN639**, which contains the backbone and tml-3' region from **pCGN986** and the two 35S promoter fragments from **pCGN164** and **pCGN638**.

**7.4.2. Construction of pCGN164**

The AluI fragment of CaMV (bp 7144-7735) (Gardner et al., Nucl.Acids Res. (1981) 9 : 2871-2888) is obtained by digestion with AluI and cloned into the HincII site of **M13mp7** (Vieira and Messing, Gene (1982) 19 : 259-268) to create **C614**. An EcoRI digest of **C614** produces the EcoRI fragment from **C614** containing the 35S promoter which is cloned into the EcoRI site of **pUC8** (Vieira and Messing, supra) to produce **pCGN146**. To trim the promoter region, the BglII site (bp 7670) is treated with BglII and Bal31 and subsequently a BglII linker is attached to the Bal31 treated DNA to produce **pCGN147**. **pCGN147** is digested with EcoRI/HphI and the resulting EcoRI-HphI fragment containing the 35S promoter is ligated into EcoRI-SmaI digested **M13mp8** (Vieira and Messing, supra) to create **pCGN164**.

7.4.3. Construction of pCGN638

Digestion of **CaMV10** (Gardner, et al., Nucl. Acids Res. (1981) 9 : 2871-2888) with BglII produces a BglII fragment containing a 35S promoter region (bp 6493-7670) which is ligated into the BamHI site of **pUCI9** (Norrander et al., Gene (1983) 26 : 101-106) to create **pCGN638**.

7.4.4. Construction of pCGN986

**pCGN986** contains a cauliflower mosaic virus 35S (CaMV35) promoter and a T-DNA tml-3' region with multiple restriction sites between them. **pCGN986** is derived from another cassette, **pCGN206**, containing a CaMV35S promoter and a different 3' region, the CaMV region VI 3'-end and pCGN971E, a tml 3' region.

**pCGN148a** containing a promoter region, selectable marker (kanamycin with 2 ATG's) and 3' region, is prepared by digesting **pCGN528** with BglII and inserting the BamHI-BglII promoter fragment from **pCGN147** (see above). This fragment is cloned into the BglII site of **pCGN528** so that the BglII site is proximal to the kanamycin gene of **pCGN528**.

The shuttle vector used for this construct **pCGN528**, is made as follows : **pCGN525** was made by digesting a plasmid containing Tn5, which harbors a kanamycin gene (Jorgensen et al., Mol. Gen. Genet. (1979) 177 : 65), with HindIII-BamHI and inserting the HindIII-BamHI fragment containing the kanamycin resistance gene into the HindIII-BamHI sites in the tetracycline gene of **pACYC184** (Chang and Cohen, J. Bacteriol. (1978) 134 : 1141-1156). **pCGN526** was made by inserting the BamHI fragment 19 of **pTiA6** (Thomashow et al., Cell (1980) 19 : 729--739) modified with XhoI linkers inserted into the SmaI site, into the BamHI site of **pCGN525**. **pCGN528** was obtained by deleting the small XhoI and religating.

**pCGN149a** is made by cloning the BamHI kanamycin gene fragment from **pMB9KanXXI** into the BamHI site of **pCGN148a**. **pMB9KanXXI** is a **pUC4K** variant (Vieira and Messing, Gene (1982) 19 : 259-268) which has the XhoI site missing but contains a function kanamycin gene from Tn903 to allow for efficient selection in Agrobacterium.

**pCGN149a** is digested with HindIII and BamHI and ligated which **pUC8** (Vieira and Messing, supra) digested with HindIII and BamHI to produce **pCGN169**. This removes the **Tn903** kanamycin marker. **pCGN565** and **pCGN169** are

both digested with HindIII and PstI and ligated to form **pCGN203**, a plasmid containing the CaMV 35S promoter and part of the 5'-end of the Tn5 kanamycin gene (up to the PstI site, (Jorgensen et al., Mol. Gen. Genet. (1979) 177 : 65). **pCGN565** is a cloning vector based on **pUC8-Cm** (K. Buckley, Ph.D. Thesis, UC San Diego 1985), but containing the polylinker from **pUC18** (Yanisch-Perron et al., Gene (1985) 53 : 103-119).

A 3' regulatory region is added to **pCGN203** from **pCGN204** (an EcoRI fragment of CaMV (bp 408-6105) containing the region VI 3' cloned into **pUC18** (Gardner et al., Nucl. Acids Res. (1981) 9 : 2871-2888) by digestion with HindIII and PstI and ligation. The resulting cassette, **pCGN206**, is the basis for the construction of **pCGN986**.

The **pTiA6** T-DNA tml 3'-sequences are subcloned from the Bam19 T-DNA fragment (Thomashow et al., Cell (1980) 19 : 729-739) as a BamHI-EcoRI fragment (nucleotides 9062 to 12, 823, numbering as in (Barker et al., Plant Mo. Biol. (1983) 2 : 335-350) and combined with the **pACYC184** (Chang and Cohen, J. Bacteriol. (1978) 134 : 1141-1156) origin of replication as an EcoRI-HindII fragment and a gentamycin resistance marker (from plasmid **pLB41**), (D. Figurski) as a BamHI-HindII fragment to produce **pCGN417**.

The unique SmaI site of **pCGN417** (nucleotide 11,207 of the Bam19 fragment) is changed to a SacI site using linkers and the BamHI-SacI fragment is subcloned into **pCGN565** to give **pCGN971**. The BamHI site of **pCGN971** is changed to an EcoRI site using linkers to yield **pCGN971E**. The resulting EcoRI-SacI fragment of **pCGN971E**, containing the tml 3' regulatory sequence is joined to **pCGN206** by digestion with EcoRI and SacI to give **pCGN975**. The small part of the Tn5 kanamycin resistance gene is deleted from the 3'-end of the CaMV 35S promoter by digestion with SalI and BglII, blunting the ends and ligating with SalI linkers. The final expression cassette **pCGN986** contains the CaMV 35S promoter followed by two SalI sites, an XbaI site, BamHI, SmaI, KpnI and the tml 3' region (nucleotides 11207-9023 of the T-DNA).

Here under are indication schemes of the constructs.

pCGN627

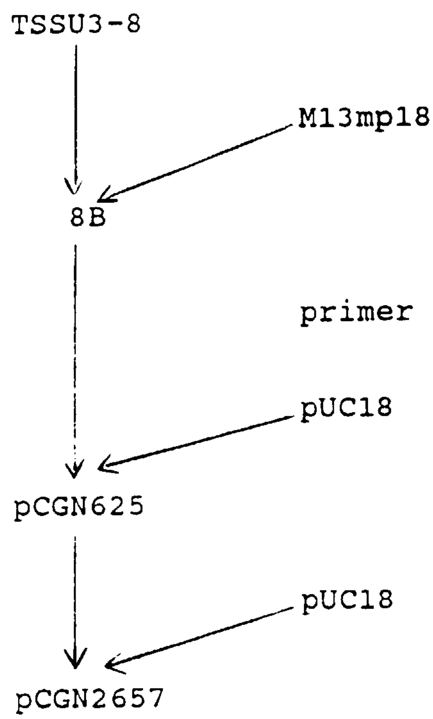

pCGN639

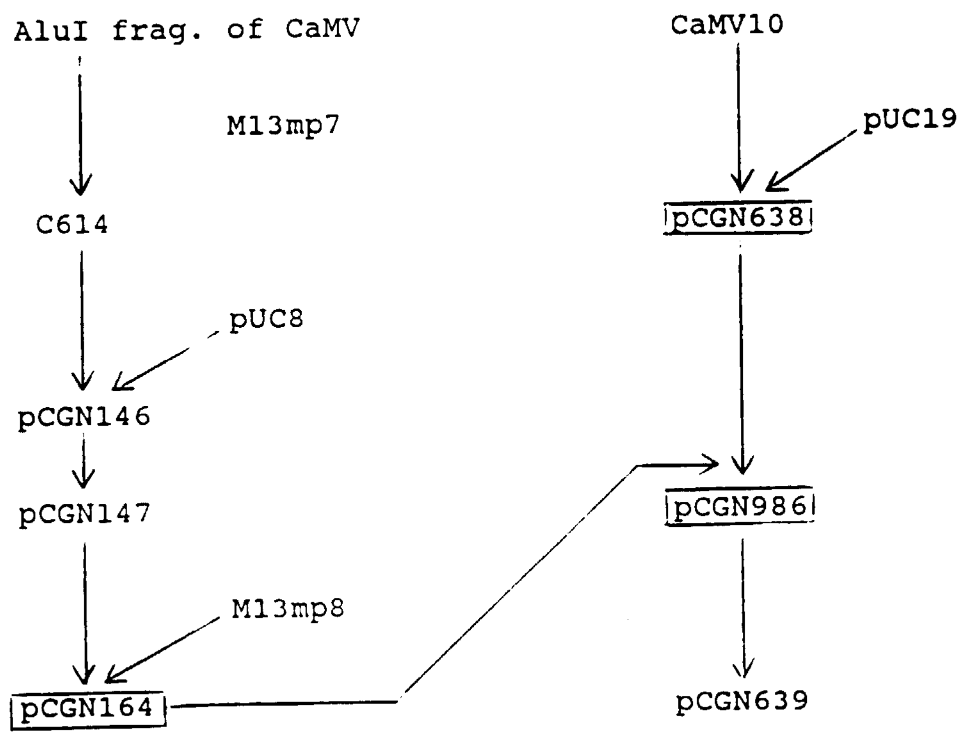
AluI frag. of CaMV
M13mp7
C614
pUC8
pCGN146
pCGN147
M13mp8
pCGN164
CaMV10
pUC19
pCGN638
pCGN986
pCGN639

pCGN986

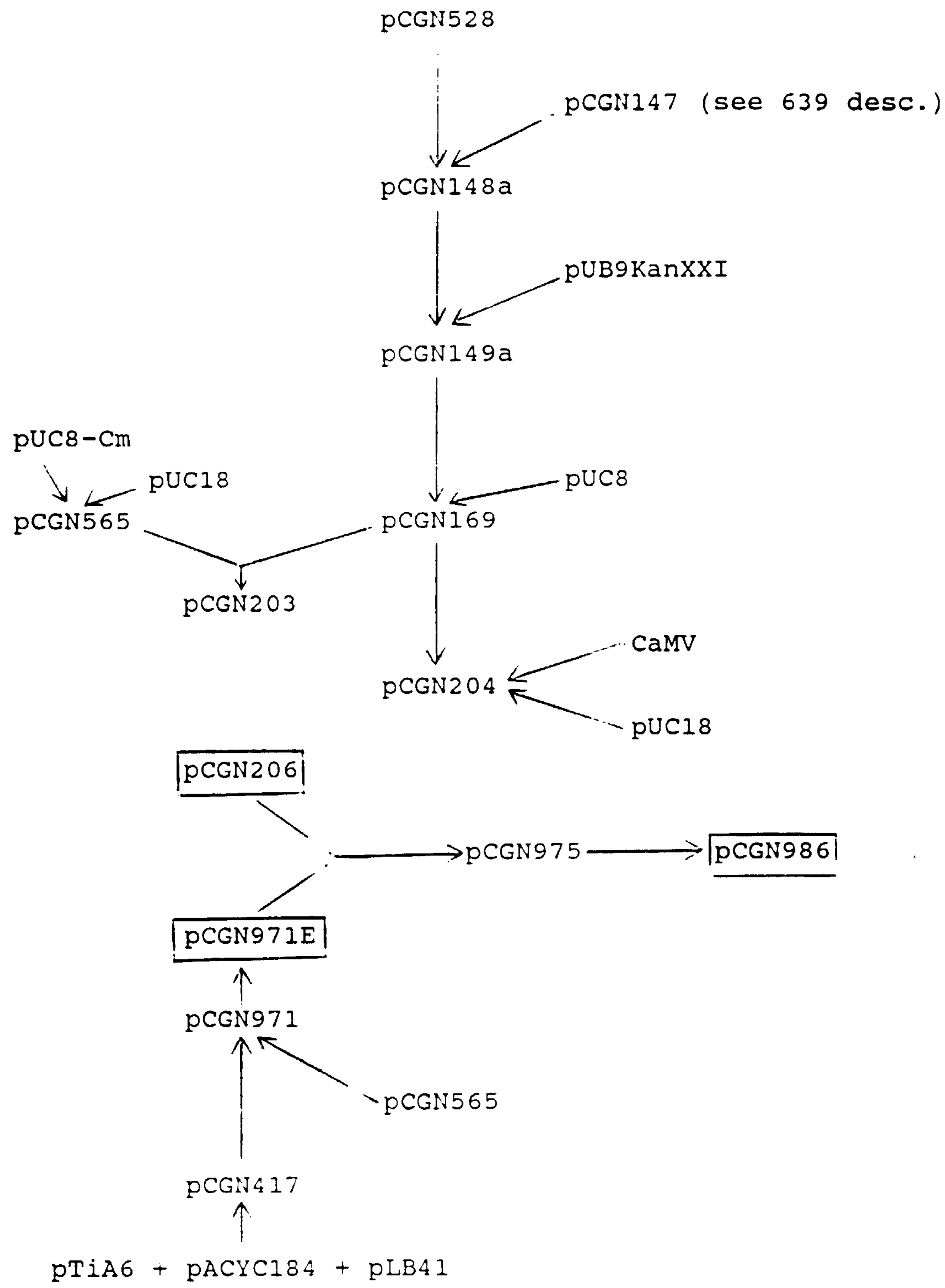


8. Transgenic SPS Tomato Plants

8.1 Production of SPS "Sense" Transgenic Tomato Plants

Tomato plants are transformed and regenerated with expression cassettes containing SPS encoding sequences (pCGN3812 and pCGN3815) via Agrobacterium tumefaciens mediated transformation (Fillatti, et al., Bio/Technology

(1987) 5:726-730). Preparation of pCGN3812, a tobacco SSU/SPS construct, and pCGN3815, a CaMV 35S/SPS construct are described in Examples 7.3 and 7.4, respectively.

8.2 Immunoblot Results

Leaves from transformed tomato plants (pCGN3812 and pCGN3815) and control tomato and corn leaves may be tested as described in Example 6.2 for SPS activity using the SPS30 and SPS90 peptide polyclonal antisera of Example 6. No cross reactivity between the antisera and the control (endogenous) tomato is seen. This indicates that the corn and tomato SPS are not highly related. As to the transgenic tomato plants, leaf extracts from plants containing the pCGN3815 or pCGN3818 constructs show signals up to levels several times that observed by the untransformed corn leaf extracts.

8.3 SPS Activity

Leaf extracts are also tested for SPS activity according to the resorcinol protocol described in Example 1.1(a). In comparison of leaf extracts from control plants and transformed tomato plants containing the SPS gene, up to 12-fold increases are observed. Higher SPS activity is also observed in some leaf extracts from transgenic tomato plants containing the corn SPS gene as compared to control corn leaf extracts.

"Tests of corn SPS in transgenic tomato leaves measured 12 hours apart (midnight/noon) show the same SPS specific activity level. Thus, SPS specific activity does not appear to be diurnally regulated in this heterologous system".

8.4 Starch and Sucrose Levels

Leaf tissue is analyzed for starch and sucrose levels according to the method of (Haissig, B.E., et al., Physiol. Plan. (1979) 47:151-157). Two controls are used, leaves from a first untransformed plant and leaves form a transformant which does not show any corn SPS immunoblot signal. The starch/sucrose levels of these two plants are essentially the same, having almost equal percentage of starch (mg/100mg dry weight) and sucrose (mg/10mg dry weight). High expressing plants containing pCGN3812 (pCGN3812-9 and pCGN3812-11) show both a reduction in leaf starch by 50% and an increase in sucrose levels by a factor of two. These data indicate that the presence of high levels of corn SPS in tomato leaves causes a modification of carbohydrate partitioning in this tissue.

Detailed description of the figures

Figure 1: shows an SDS-PAGE profile at various stages of SPS purification and the quality of the final preparation.

Figure 2: shows the results of a Western analysis of SPS using monoclonal antibodies.

Figure 3 : shows peptide sequences (SEQ ID NOS: 1-5) derived from SPS protein. All peptides are typed N -> C terminal.

Figure 4 : shows oligonucleotides used for the PCR reactions CD3 and CD4 in relation to the peptides (antisense sequences are presented upside down). Arrows point to the direction the oligonucleotides will prime the polymerase.

Figure 5A : shows agarose gel electrophoresis of CD3 and CD4 PCR reactions. The sizes are given in kb.

Figure 5B : shows autoradiograph of Southern blot of CD3 and CD4 PCF reactions probed with oligonucleotides 4k5 (SEQ ID NO: 1-3).

Figure 6 : shows schematic diagrams representing SPS cDNA and selected clones. The upper bar represents the entire 3509 bp combined map. Translation stop and start are indicated.

Figure 7 : shows the assembled SPS cDNA sequence. The sequences of clones SPS 90, SPS 61 and SPS 3 were fused at the points indicated in Fig. 2. The SPS reading frame is translated. All SPS protein derived peptide sequences are indicated.

Figure 8 : shows Western blots showing characteristics of rabbit SPS 90 and SPS 30 antisera. pAS** = preimmune serum, SPS 30 rabbit ; AS** = immune serum anti SPS 90. Molecular weight markers at left, where indicated. S = SPS 120 kd polypeptide ; S* = SPS 90 kd polypeptide ; S** = SPS 30 kd polypeptide.

Figure 9A : shows a Comassie Blue-stained gel of total protein isolated from a 30 day old corn plant. M = size marker ; R = roots ; 1-8 = leaf numbers counting from the bottom of the plant. Leaf 5 has been cut into 5 segments from the leaf tip (5a) to the end of the sheath (5c). PEP = phosphoenolpyruvate carboxylase.

Figure 9B : shows the results of Western blot analysis using a mixture of antiSPS 30 and antiSPS 90 antisera against total plant protein isolated from a 30 day old corn plant. The signal corresponding to SPS appears at 120-140 kd.

## Claims

1. Homogenous protein being capable of catalyzing the formation of sucrose phosphate wherein said protein is obtainable from corn.

2. Homogenous protein wherein said protein is a sucrose phosphate synthase (SPS) having fructose-6-phosphate and UDP-glucose substrates.

3. The protein of Claim 1 comprising a corn SPS having a molecular weight from 100 to 130 kd, and a specific activity of greater than 0,05 U per mg (1 U corresponds to 1 micromole of sucrose formed per minute per mg of protein at 37°C).

4. The protein of Claim 3 comprising a specific activity of 25U per mg.

5. The protein of Claim 1 comprising an amino acid sequence according to Fig. 7.

6. Protein derivatives from the native SPS proteins defined in any of claims 1 to 5 modified by genetic engeneering and having the SPS activity.

7. Process to prepare proteins defined in claim 1, characterized in that :

   **a)** one extracts from parts containing SPS, preserved at low temperature, by grinding, centrifugation and filtration,
   **b)** one increases the SPS rate of the extract so obtained by precipitation in an appropriate solvent, centrifugation and solubilization of the precipitate in a buffer solution.
   **c)** one purifies the protein so obtained by chromatography and if desired,
   **d)** one prepares the hybridomas, and monoclonal antibodies from an antigenic solution prepared from one of the preparations a, b, c,
   **e)** one screens the hybridomas and raises monoclonal antibodies specifically directed against SPS,
   **f)** one purifies the SPS so obtained with the monoclonal antibodies so prepared.

8. Process of preparation of corn SPS according to claim 7, characterized in that :

   **a)** one extracts from part of corn plants by grinding centrifugation and filtration,
   **b)** one increases the SPS rate of the extract so obtained by precipitation in polyethyleneglycol, centrifugation and solubilization of the precipitate obtained in a buffer solution,
   **c)** one purifies the protein obtained in low pression anion exchange chromatography and in chromatography on heparin sepharose, then in anion exchange high performance chromatography,
   **d)** one purifies the active pools from step c by passage on 2 high performance chromatography columns, and if desired,
   **e)** one prepares the hybridomas and monoclonal antibodies from an antigenic solution prepared from one preparation a, b, c,
   **f)** one screens the hybridomas and isolates the monoclonal antibodies specifically directed against SPS,
   **g)** one purifies the SPS preparation with the monoclonal antibodies so obtained.

9. Lines of hybridoma cells obtained in claim 7 or 8.

10. Lines of hybridoma cells according to claim 9 deposed according to the European Convention in Institut Pasteur in Paris, and described as :

| | |
|---|---|
| SPA 2-2-3 : I-971 | SPB 3-2-19 : I-973 |
| SPA 2-2-22 : I-970 | SPB 5-2-10 : I-974 |
| SPA 2-2-25 : I-972 | SPB 5-4-2 : I-975 |
| | SPB 13-1-7 : I-976 |
| | SPB 13-2-2 : I-977 |

11. Monoclonal antibodies specifically directed against proteins prepared according to claims 1 to 6.

12. Monoclonal antibodies according to claim 11 specifically directed against corn SPS.

13. Process of preparation of proteins according to any of claims 1 to 6, characterized in that a preparation containing the proteins is purified on a chromatography column having monoclonal antibodies according to claims 11 and 12 specifically raised against the proteins.

14. An extrachromosomal DNA sequence comprising sequence encoding a sucrose phosphate synthase (SPS).

15. The sequence of Claim 14 obtainable from corn.

16. The sequence of Claim 14 comprising cDNA.

**Patentansprüche**

1. Homogenes Protein, das die Bildung von Saccharosephosphat katalysieren kann, wobei das Protein aus Mais erhältlich ist.

2. Homogenes Protein, wobei das Protein eine Saccharosephosphatsynthetase (SPS) mit Fructose-6-phosphat und UDP-Glucose als Substrate ist.

3. Protein nach Anspruch 1, das eine Mais -SPS mit einem Molekulargewicht von 100 bis 130 kD und einer spezifischen Aktivität von über 0,05 U/mg ist (1U entspricht 1 µmol gebildete Saccharose / Minute / mg Protein bei 37°C).

4. Protein nach Anspruch 3, das eine spezifische Aktivität von 25 U/mg aufweist.

5. Protein nach Anspruch 1, das eine Aminosäuresequenz gemäß Fig. 7 umfaßt.

6. Proteinderivate der nativen SPS-Proteine nach einem der Ansprüche 1 bis 5, die mittels Gentechnologie modifiziert wurden und SPS-Aktivität besitzen.

7. Verfahren zur Herstellung von Proteinen nach Anspruch 1, dadurch gekennzeichnet, daß:

   a) mit bei tiefer Temperatur aufbewahrten, SPS-enthaltenen Teilen durch Mahlen, Zentrifugation und Filtration eine Extraktion durchgeführt wird,
   b) die SPS-Menge des so erhaltenen Extrakts durch Ausfällung in einem geeigneten Lösungsmittel, Zentrifugation und Solubilisierung des Ausgefällten in einer Pufferlösung erhöht wird,
   c) das so erhaltene Protein mittels Chromatographie gereinigt wird,

   und nach Wunsch

   d) aus einer antigenen Lösung, die aus einer der Zubereitungen a, b, c hergestellt wurde, Hybridomas und monoclonale Antikörper hergestellt werden,
   e) die Hybridomas durchmustert und spezifisch gegen SPS gerichtete, monoclonale Antikörper erzeugt werden,
   f) das so erhaltene SPS mit den so hergestellten monoclonalen Antikörpern gereinigt wird.

8. Verfahren zur Herstellung von Mais -SPS nach Anspruch 7, dadurch gekennzeichnet, daß:

   a) mit einem Teil von Maispflanzen durch Mahlen, Zentrifugation und Filtration eine Extraktion durchgeführt wird,
   b) die SPS-Menge des so erhaltenen Extrakts durch Ausfällung in Polyethylenglycol, Zentrifugation und Solubilisierung des erhaltenen Ausgefällten in einer Pufferlösung erhöht wird,
   c) das erhaltene Protein mittels Niederdruck-Anionen austauscherchromatographie und Chromatographie auf Heparin-Sepharose und anschließend mittels Anionen austauscher-Hochleistungschromatographie gereinigt wird,
   d) die aktiven Pools aus Schritt c durch Leiten durch 2 Hochleistungschromatographiesäulen gereinigt werden,

und nach Wunsch

d) aus einer antigenen Lösung, die aus einer der Zubereitungen a, b, c hergestellt wurde, Hybridomas und monoclonale Antikörper hergestellt werden,
e) die Hybridomas durchmustert und spezifisch gegen SPS gerichtete, monoclonale Antikörper erzeugt werden,
f) die SPS-Zubereitung mit den so erhaltenen monoclonalen Antikörpern gereinigt wird.

**9.** Hybridomazellinien, die gemäß Anspruch 7 oder 8 erhalten werden.

**10.** Hybridomazellinien nach Anspruch 9, die gemäß dem Europäischen Vertrag in dem Pasteur Institut in Paris hinterlegt wurden und bezeichnet werden als:

| | |
|---|---|
| SPA 2-2-3 : I-971 | SPB 3-2-19 : I-973 |
| SPA 2-2-22 : I-970 | SPB 5-2-10 : I-974 |
| SPA 2-2-25 : I-972 | SPB 5-4-2 : I-975 |
| | SPB 13-1-7 : I-976 |
| | SPB 13-2-2 : I-977 |

**11.** Monoclonale Antikörper, die spezifisch gegen nach einem der Ansprüche 1 bis 6 hergestellte Proteine gerichtet sind.

**12.** Monoclonale Antikörper nach Anspruch 11, die spezifisch gegen Mais -SPS gerichtet sind.

**13.** Verfahren zur Herstellung von Proteinen nach einem der Ansprüche 1 bis 6, dadurch gekennzeichnet, daß eine die Proteine enthaltende Zubereitung auf einer Chromatographiesäule gereingt wird, die monoclonale Antikörper gemäß den Ansprüchen 1 und 12, die spezifisch gegen die Proteine erzeugt wurden, enthält.

**14.** Extrachromosomale DNA-Sequenz, die eine Saccharosephoshatsynthetase (SPS) codierende Sequenz enthält.

**15.** Sequenz nach Anspruch 14, die aus Mais erhältlich ist.

**16.** Sequenz nach Anspruch 14, die cDNA umfaßt.

## Revendications

**1.** Protéine homogène susceptible de catalyser la formation de phosphate de saccharose, caractérisée en ce que ladite protéine peut être obtenue du maïs.

**2.** Protéine homogène, caractérisée en ce que ladite protéine est une saccharose phosphate synthase (SPS) ayant comme substrats le fructose-6-phosphate et l'UDP-glucose.

**3.** Protéine selon la revendication 1, comprenant une SPS de maïs ayant un poids moléculaire de 100 à 130 kd, et une activité spécifique supérieure à 0,05 U par mg (1 U correspond à 1 micromole de saccharose formé par minute par mg de protéine à 37°C).

**4.** Protéine selon la revendication 3, présentant une activité spécifique de 25 U par mg.

**5.** Protéine selon la revendication 1, comprenant une séquence d'acides aminés selon la fig. 7.

**6.** Dérivés protéiques issus des protéines SPS natives définies selon l'une quelconque des revendications 1 à 5, modifiés par génie génétique et ayant l'activité de SPS.

**7.** Procédé de préparation de protéines défini selon la revendication 1, caractérisé en ce que:

**a)** on extrait de parties contenant la SPS, conservées à basse température, par broyage, centrifugation et

filtration,
**b)** on augmente le taux de SPS de l'extrait ainsi obtenu par précipitation dans un solvant approprié, centrifugation et solubilisation du précipité dans une solution tampon,
**c)** on purifie la protéine ainsi obtenue par chromatographie et si on le souhaite,
**d)** on prépare les hybridomes, et les anticorps monoclonaux à partir d'une solution antigénique préparée à partir de l'une des préparations a, b, c,
**e)** on réalise le criblage des hybridomes et on dresse des anticorps monoclonaux dirigés spécifiquement contre la SPS,
**f)** on purifie la SPS ainsi obtenue avec les anticorps monoclonaux ainsi préparés.

**8.** Procédé de préparation de SPS de maïs selon la revendication 7, caractérisé en ce que:

**a)** on extrait d'une partie de plantes de maïs par broyage, centrifugation et filtration,
**b)** on augmente le taux de SPS de l'extrait ainsi obtenu par précipitation dans le polyéthylèneglycol, centrifugation et solubilisation du précipité obtenu dans une solution tampon,
**c)** on purifie la protéine obtenue par chromatographie échangeuse d'anions à basse pression et par chromatographie sur héparine-Sépharose, puis par chromatographie échangeuse d'anions à haute performance ,
**d)** on purifie les fractions actives réunies de l'étape c par passage sur 2 colonnes de chromatographie haute performance, et si on le souhaite,
**e)** on prépare les hybridomes, et les anticorps monoclonaux à partir d'une solution antigénique préparée à partir de l'une des préparations a, b, c,
**f)** on réalise le criblage des hybridomes et on isole les anticorps monoclonaux dirigés spécifiquement contre la SPS,
**g)** on purifie la préparation de SPS à l'aide des anticorps monoclonaux ainsi obtenus.

**9.** Lignées cellulaires d'hybridome obtenues selon la revendication 7 ou 8.

**10.** Lignées cellulaires d'hybridome selon la revendication 9, déposées conformément à la Convention Européenne auprès de l'Institut Pasteur à Paris, et décrites comme:

| | |
|---|---|
| SPA 2-2-3 : I-971 | SPB 3-2-19 : I-973 |
| SPA 2-2-22 : I-970 | SPB 5-2-10 : I-974 |
| SPA 2-2-25 : I-972 | SPB 5-4-2 : I-975 |
| | SPB 13-1-7 : I-976 |
| | SPB 13-2-2 : I-977 |

**11.** Anticorps monoclonaux dirigés spécifiquement contre les protéines préparées selon les revendications 1 à 6.

**12.** Anticorps monoclonaux selon la revendication 11, dirigés spécifiquement contre la SPS de maïs.

**13.** Procédé de préparation des protéines selon l'une quelconque des revendications 1 à 6, caractérisé en ce qu'une préparation contenant les protéines est purifiée sur une colonne chromatographique ayant des anticorps monoclonaux selon les revendications 11 et 12 dressés spécifiquement contre les protéines.

**14.** Séquence d'ADN extrachromosomique comprenant une séquence codant pour une saccharose phosphate synthase (SPS).

**15.** Séquence selon la revendication 14, pouvant être obtenue du maïs.

**16.** Séquence selon la revendication 14, comprenant l'ADNc.

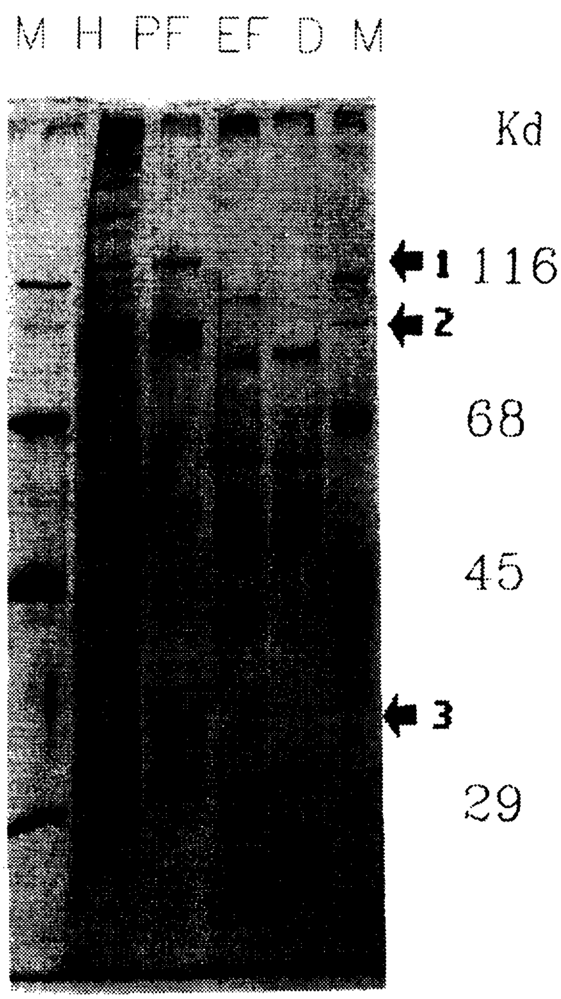


FIGURE 1

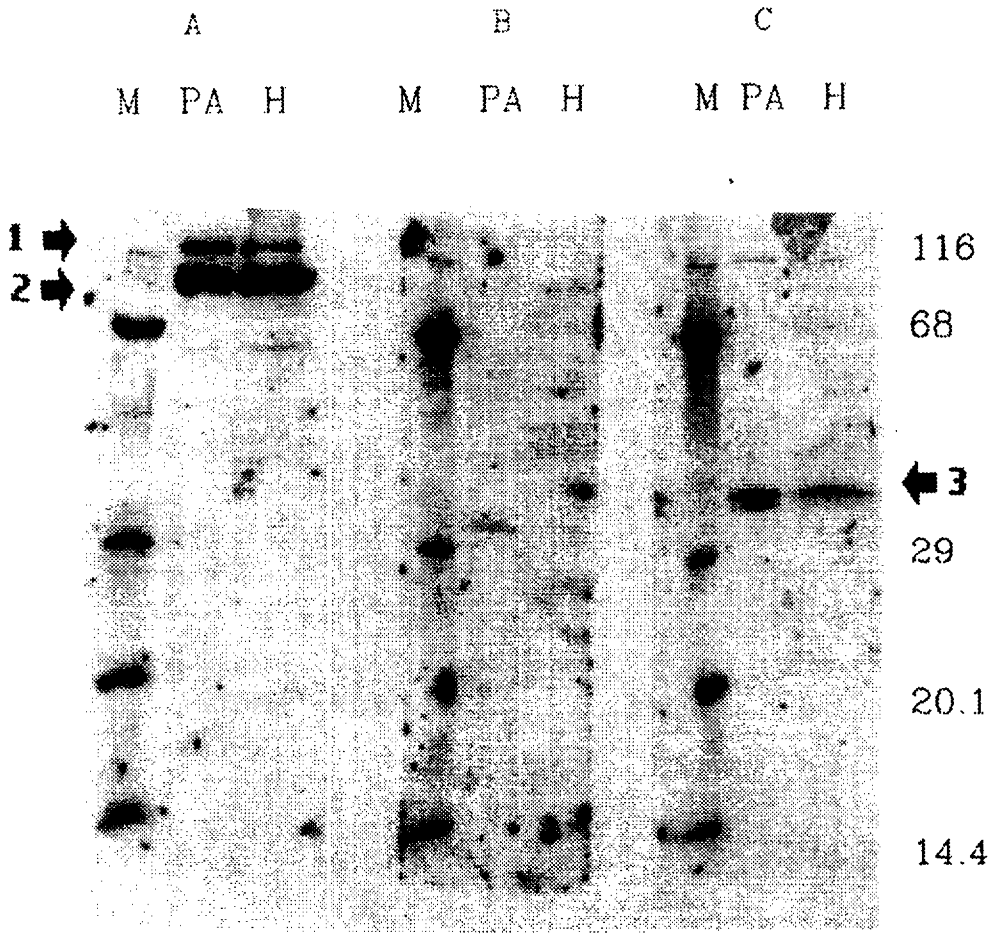


FIGURE 2

SPS 90 kd PEPTIDES

A8 ThrTrpIleLys

B4 TyrValValGluLeuAlaArg

B11 SerMetProProIleTrpAlaGluValMetArg

SPS 30 kd PEPTIDES

4K LeuArgProAspGlnAspTyrLeuMetHisIleSerHisArg

12N TrpSerHisAspGlyAlaArg

FIGURE 3

```
           B11                                            4K
           SerMetProProIleTrpAlaGluValMetArg          LeuArgProAspGlNAspTyrLeuMetHisIleSerHisArg
           TCAATGCCACCAATATGGGCAGAAGTAATGCGA          TTAAGACCAGACCAAGACTACTTAATGCACATAAGACACAGA
           AGC     C  C  C     C  G  C   A C          C GC C  C  T  G  T  TC C     T  CTCC  TC C
             G     G  G  T     G     G     G            C  G  G              G        T  G     G
             T     T  T        T     T     T            T  T  T              T           T     T

CD3 B11C     ATGCCICCIATATGGGCIGA --->                <---     CTAGTCCTAATAGAITACGT            4K3
                  C  C  C     C                                  G  T  G  G  C
                        T

CD4 B11B <-- TACGGIGGITAAACCCGICT               GACCAAGACTACCTIATGCA ----->                    4K1S
                  C  C  G     C                   T  G  T  T  C
                        T

                         ACCCGICTCCAITACGC   B11A              TACGTATAATCAGTAGC               4K2
                              C  T  C   T                           G  G  G  GT
                                                                       T

                                                  AAIGCIGGICTAGT                               4K5
                                                  G CT C  C  G
```

FIGURE 4

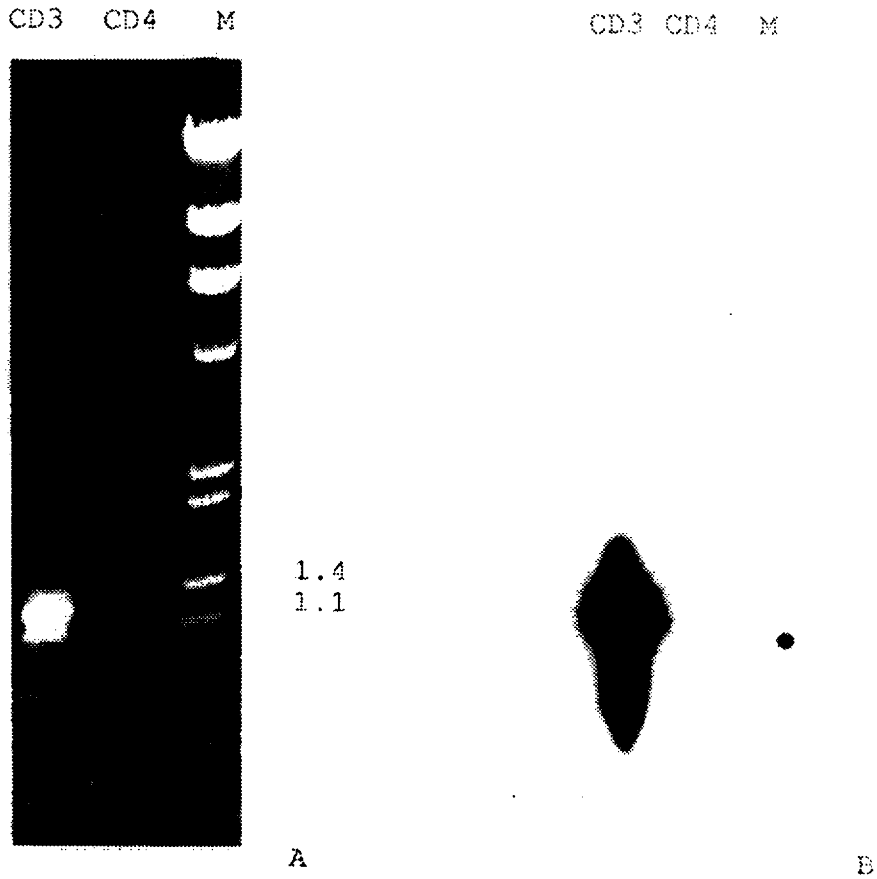


FIGURE 5

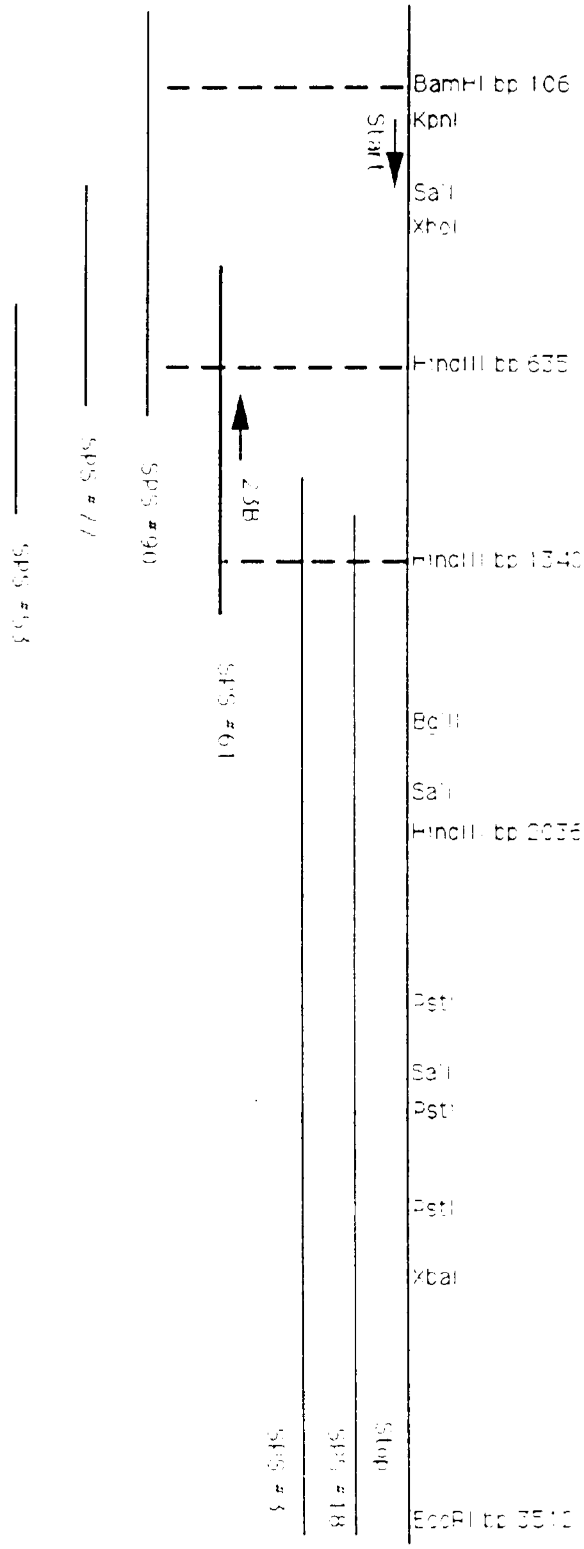
BamHI bp 106
KpnI
SalI
XhoI
HincIII bp 635
HincIII bp 1340
BglII
SalI
HincIII bp 2036
PstI
SalI
PstI
PstI
XbaI
EcoRI bp 3512
Start
Stop
SPS #3
SPS #77
SPS #90
SPS #61
SPS #5
SPS #18

FIGURE 6

SPS cDNA sequence

```
     EcoRI
     |
  1  GAATTCCGGCGTGGGCGCTGGGCTAGTGCTCCCGCAGCGAGCGATCTGAGAGAACGGTAGAGTTCCGGC   69
     2

                                                BamHI
                                                |
 70  CGGGCGCGCGGGAGAGGAGGAGGGTCGGGCGGGGAGGATCCGATGGCCGGGAACGAGTGGATCAATGGG  138
                                                      METAlaGlyAsnGluTrpIleAsnGly
                                                106

       KpnI
       |
139  TACCTGGAGGCGATCCTCGACAGCCACACCTCGTCGCGGGGTGCCGGCGGCGGCGGCGGCGGGGGGGAC  207
     TyrLeuGluAlaIleLeuAspSerHisThrSerSerArgGlyAlaGlyGlyGlyGlyGlyGlyGlyAsp
       142

208  CCCAGGTCGCCGACGAAGGCGGCGAGCCCCCGCGGCGCGCACATGAACTTCAACCCCTCGCACTACTTC  276
     ProArgSerProThrLysAlaAlaSerProArgGlyAlaHisMETAsnPheAsnProSerHisTyrPhe

                                  SalI
                                  |
277  GTCGAGGAGGTGGTCAAGGGCGTCGACGAGAGCGACCTCCACCGGACGTGGATCAAGGTCGTCGCCACC  345
     ValGluGluValValLysGlyValAspGluSerAspLeuHisArgThrTrpIleLysValValAlaThr
                                  299                         A8

                                       XhoI
                                       |
346  CGCAACGCCCGCGAGCGCAGCACCAGGCTCGAGAACATGTGCTGGCGGATCTGGCACCTCGCGCGCAAG  414
     ArgAsnAlaArgGluArgSerThrArgLeuGluAsnMETCysTrpArgIleTrpHisLeuAlaArgLys
                                       374

415  AAGAAGCAGCTGGAGCTGGAGGGCATCCAGAGAATCTCGGCAAGAAGGAAGGAACAGGAGCAGGTGCGT  483
     LysLysGlnLeuGluLeuGluGlyIleGlnArgIleSerAlaArgArgLysGluGlnGluGlnValArg

484  CGTGAGGCGACGGAGGACCTGGCCGAGGATCTGTCAGAAGGCGAGAAGGGAGACACCATCGGCGAGCTT  552
     ArgGluAlaThrGluAspLeuAlaGluAspLeuSerGluGlyGluLysGlyAspThrIleGlyGluLeu

553  GCGCCGGTTGAGACGACCAAGAAGAAGTTCCAGAGGAACTTCTCTGACCTTACCGTCTGGTCTGACGAC  621
     AlaProValGluThrThrLysLysLysPheGlnArgAsnPheSerAspLeuThrValTrpSerAspAsp

                 HindIII
                 |
622  AATAAGGAGAAGAAGCTTTACATTGTGCTCATCAGCGTGCATGGTCTTGTTCGTGGAGAAAACATGGAA  690
     AsnLysGluLysLysLeuTyrIleValLeuIleSerValHisGlyLeuValArgGlyGluAsnMETGlu
                 635

691  CTAGGTCGTGATTCTGATACAGGTGGCCAGGTGAAATATGTGGTCGAACTTGCAAGAGCGATGTCAATG  759
     LeuGlyArgAspSerAspThrGlyGlyGlnValLysTyrValValGluLeuAlaArgAlaMETSerMET
                                                        B4

760  ATGCCTGGAGTGTACAGGGTGGACCTCTTCACTCGTCAAGTGTCATCTCCTGACGTGGACTGGAGCTAC  828
     METProGlyValTyrArgValAspLeuPheThrArgGlnValSerSerProAspValAspTrpSerTyr
```

FIGURE 7 1/4

829 GGTGAGCCAACCGAGATGTTATGCGCCGGTTCCAATGATGGAGAGGGGATGGGTGAGAGTGGCGGAGCC 897
GlyGluProThrGluMETLeuCysAlaGlySerAsnAspGlyGluGlyMETGlyGluSerGlyGlyAla

898 TACATTGTGCGCATACCGTGTGGGCCGCGGGATAAATACCTCAAGAAGGAAGCGTTGTGGCCTTACCTC 966
TyrIleValArgIleProCysGlyProArgAspLysTyrLeuLysLysGluAlaLeuTrpProTyrLeu

967 CAAGAGTTTGTCGATGGAGCCCTTGCGCATATCCTGAACATGTCCAAGGCTCTGGGAGAGCAGGTTGGA 1035
GlnGluPheValAspGlyAlaLeuAlaHisIleLeuAsnMETSerLysAlaLeuGlyGluGlnValGly

1036 AATGGGAGGCCAGTACTGCCTTACGTGATACATGGGCACTATGCCGATGCTGGAGATGTTGCTGCTCTC 1104
AsnGlyArgProValLeuProTyrValIleHisGlyHisTyrAlaAspAlaGlyAspValAlaAlaLeu

1105 CTTTCTGGTGCGCTGAATGTGCCAATGGTGCTCACTGGCCACTCACTTGGGAGGAACAAGCTGGAACAA 1173
LeuSerGlyAlaLeuAsnValProMETValLeuThrGlyHisSerLeuGlyArgAsnLysLeuGluGln

1174 CTGCTGAAGCAAGGGCGCATGTCCAAGGAGGAGATCGATTCGACATACAAGATCATGAGGCGTATCGAG 1242
LeuLeuLysGlnGlyArgMETSerLysGluGluIleAspSerThrTyrLysIleMETArgArgIleGlu

1243 GGTGAGGAGCTGGCCCTGGATGCGTCAGAGCTTGTAATCACGAGCACAAGGCAGGAGATTGATGAGCAG 1311
GlyGluGluLeuAlaLeuAspAlaSerGluLeuValIleThrSerThrArgGlnGluIleAspGluGln

HindIII

1312 TGGGGATTGTACGATGGATTTGATGTCAAGCTTGAGAAAGTGCTGAGGGCACGGGCGAGGCGCGGGGTT 1380
TrpGlyLeuTyrAspGlyPheAspValLysLeuGluLysValLeuArgAlaArgAlaArgArgGlyVal
1340

NcoI

1381 AGCTGCCATGGTCGTTACATGCCTAGGATGGTGGTGATTCCTCCGGGAATGGATTTCAGCAATGTTGTA 1449
SerCysHisGlyArgTyrMETProArgMETValValIleProProGlyMETAspPheSerAsnValVal
1387

1450 GTTCATGAAGACATTGATGGGGATGGTGACGTCAAAGATGATATCGTTGGTTTGGAGGGTGCCTCACCC 1518
ValHisGluAspIleAspGlyAspGlyAspValLysAspAspIleValGlyLeuGluGlyAlaSerPro

1519 AAGTCAATGCCCCCAATTTGGGCCGAAGTGATGCGGTTCCTGACCAACCCTCACAAGCCGATGATCCTG 1587
LysSerMETProProIleTrpAlaGluValMETArgPheLeuThrAsnProHisLysProMETIleLeu
**B11**

1588 GCGTTATCAAGACCAGACCCGAAGAAGAACATCACTACCCTCGTCAAAGCGTTTGGAGAGTGTCGTCCA 1656
AlaLeuSerArgProAspProLysLysAsnIleThrThrLeuValLysAlaPheGlyGluCysArgPro

1657 CTCAGGGAACTTGCAAACCTTACTCTGATCATGGGTAACAGAGATGACATCGACGACATGTCTGCTGGC 1725
LeuArgGluLeuAlaAsnLeuThrLeuIleMETGlyAsnArgAspAspIleAspAspMETSerAlaGly

1726 AATGCCAGTGTCCTCACCACAGTTCTGAAGCTGATTGACAAGTATGATCTGTACGGAAGCGTGGCGTTC 1794
AsnAlaSerValLeuThrThrValLeuLysLeuIleAspLysTyrAspLeuTyrGlySerValAlaPhe

BglII

1795 CCTAAGCATCACAATCAGGCTGACGTCCCGGAGATCTATCGCCTCGCGGCCAAAATGAAGGGCGTCTTC 1863
ProLysHisHisAsnGlnAlaAspValProGluIleTyrArgLeuAlaAlaLysMETLysGlyValPhe
1827

1864 ATCAACCCTGCTCTCGTTGAGCCGTTTGGTCTCACCCTGATCGAGGCTGCGGCACACGGACTCCCGATA 1932
IleAsnProAlaLeuValGluProPheGlyLeuThrLeuIleGluAlaAlaAlaHisGlyLeuProIle

FIGURE 7 2/4

```
                             SalI
                             |
1933 GTCGCTACCAAGAATGGTGGTCCGGTCGACATTACAAATGCATTAAACAACGGACTGCTCGTTGACCCA 2001
     ValAlaThrLysAsnGlyGlyProValAspIleThrAsnAlaLeuAsnAsnGlyLeuLeuValAspPro
                             1958

                                        HindIII
                                        |
2002 CACGACCAGAACGCCATCGCTGATGCACTGCTGAAGCTTGTGGCAGACAAGAACCTGTGGCAGGAATGC 2070
     HisAspGlnAsnAlaIleAlaAspAlaLeuLeuLysLeuValAlaAspLysAsnLeuTrpGlnGluCys
                                        2036

2071 CGGAGAAACGGGCTGCGCAACATCCACCTCTACTCATGGCCGGAGCACTGCCGCACTTACCTCACCAGG 2139
     ArgArgAsnGlyLeuArgAsnIleHisLeuTyrSerTrpProGluHisCysArgThrTyrLeuThrArg

2140 GTGGCCGGGTGCCGGTTAAGGAACCCGAGGTGGCTGAAGGACACACCAGCAGATGCCGGAGCCGATGAG 2208
     ValAlaGlyCysArgLeuArgAsnProArgTrpLeuLysAspThrProAlaAspAlaGlyAlaAspGlu

                        NcoI
                        |
2209 GAGGAGTTCCTGGAGGATTCCATGGACGCTCAGGACCTGTCACTCCGTCTGTCCATCGACGGTGAGAAG 2277
     GluGluPheLeuGluAspSerMETAspAlaGlnAspLeuSerLeuArgLeuSerIleAspGlyGluLys
                        2229

2278 AGCTCGCTGAACACTAACGATCCACTGTGGTTCGACCCCCAGGATCAAGTGCAGAAGATCATGAACAAC 2346
     SerSerLeuAsnThrAsnAspProLeuTrpPheAspProGlnAspGlnValGlnLysIleMETAsnAsn

2347 ATCAAGCAGTCGTCAGCGCTTCCTCCGTCCATGTCCTCAGTCGCAGCCGAGGGCACAGGCAGCACCATG 2415
     IleLysGlnSerSerAlaLeuProProSerMETSerSerValAlaAlaGluGlyThrGlySerThrMET

2416 AACAAATACCCACTCCTGCGCCGGCGCCGGCGCTTGTTCGTCATAGCTGTGGACTGCTACCAGGACGAT 2484
     AsnLysTyrProLeuLeuArgArgArgArgArgLeuPheValIleAlaValAspCysTyrGlnAspAsp

                              PstI
                              |
2485 GGCCGTGCTAGCAAGAAGATGCTGCAGGTGATCCAGGAAGTTTTCAGAGCAGTCCGATCGGACTCCCAG 2553
     GlyArgAlaSerLysLysMETLeuGlnValIleGlnGluValPheArgAlaValArgSerAspSerGln
                              2511

            BglII             SalI                                      PstI
            |                 |                                         |
2554 ATGTTCAAGATCTCAGGGTTCACGCTGTCGACTGCCATGCCGTTGTCCGAGACACTCCAGCTTCTGCAG 2622
     METPheLysIleSerGlyPheThrLeuSerThrAlaMETProLeuSerGluThrLeuGlnLeuLeuGln
            2562              2581                                      2622

2623 CTCGGCAAGATCCCAGCGACCGACTTCGACGCCCTCATCTGTGGCAGCGGCAGCGAGGTGTACTATCCT 2691
     LeuGlyLysIleProAlaThrAspPheAspAlaLeuIleCysGlySerGlySerGluValTyrTyrPro

2692 GGCACGGCGAACTGCATGGACGCTGAAGGAAAGCTGCGCCCAGATCAGGACTATCTGATGCACATCAGC 2760
     GlyThrAlaAsnCysMETAspAlaGluGlyLysLeuArgProAspGlnAspTyrLeuMETHisIleSer
                                                         4K

2761 CACCGCTGGTCCCATGACGGCGCGAGGCAGACCATAGCGAAGCTCATGGGCGCTCAGGACGGTTCAGGC 2829
     HisArgTrpSerHisAspGlyAlaArgGlnThrIleAlaLysLeuMETGlyAlaGlnAspGlySerGly
           =====================
                 12N
```

FIGURE 7  3/4

```
2830 GACGCTGTCGAGCAGGACGTGGCGTCCAGTAATGCACACTGTGTCGCGTTCCTCATCAAAGACCCCCAA 2898
     AspAlaValGluGlnAspValAlaSerSerAsnAlaHisCysValAlaPheLeuIleLysAspProGln

2899 AAGGTGAAAACGGTCGATGAGATGAGGGAGCGGCTGAGGATGCGTGGTCTCCGCTGCCACATCATGTAC 2967
     LysValLysThrValAspGluMETArgGluArgLeuArgMETArgGlyLeuArgCysHisIleMETTyr

         PstI
         |
2968 TGCAGGAACTCGACAAGGCTTCAGGTTGTCCCTCTGCTAGCATCAAGGTCACAGGCACTCAGGTATCTT 3036
     CysArgAsnSerThrArgLeuGlnValValProLeuLeuAlaSerArgSerGlnAlaLeuArgTyrLeu
         2972

                                                                     XbaI
                                                                     |
3037 TCCGTGCGCTGGGGCGTATCTGTGGGGAACATGTATCTGATCACCGGGGAACATGGCGACACCGATCTA 3105
     SerValArgTrpGlyValSerValGlyAsnMETTyrLeuIleThrGlyGluHisGlyAspThrAspLeu
                                                                     3103

3106 GAGGAGATGCTATCCGGGCTACACAAGACCGTGATCGTCCGTGGCGTCACCGAGAAGGGTTCGGAAGCA 3174
     GluGluMETLeuSerGlyLeuHisLysThrValIleValArgGlyValThrGluLysGlySerGluAla

3175 CTGGTGAGGAGCCCAGGAAGCTACAAGAGGGACGATGTCGTCCCGTCTGAGACCCCCTTGGCTGCGTAC 3243
     LeuValArgSerProGlySerTyrLysArgAspAspValValProSerGluThrProLeuAlaAlaTyr

3244 ACGACTGGTGAGCTGAAGGCCGACGAGATCATGCGGGCTCTGAAGCAAGTCTCCAAGACTTCCAGCGGC 3312
     ThrThrGlyGluLeuLysAlaAspGluIleMETArgAlaLeuLysGlnValSerLysThrSerSerGly

3313 ATGTGAATTTGATGCTTCTTTTACATTTTGTCCTTTTCTTCACTGCTATATAAAATAAGTTGTGAACAG 3381
     MET .

3382 TACCGCGGGTGTGTATATATATATTGCAGTGACAAATAAAACAGGACACTGCTAACTATACTGGTGAAT 3450

                                                      EcoRI
                                                      |
3451 ATACGACTGTCAAGATTGTATGCTAAGTACTCCATTTCTCAATGTATCAATCGGAATTC 3509
                                                      3505
```

FIGURE 7 4/4

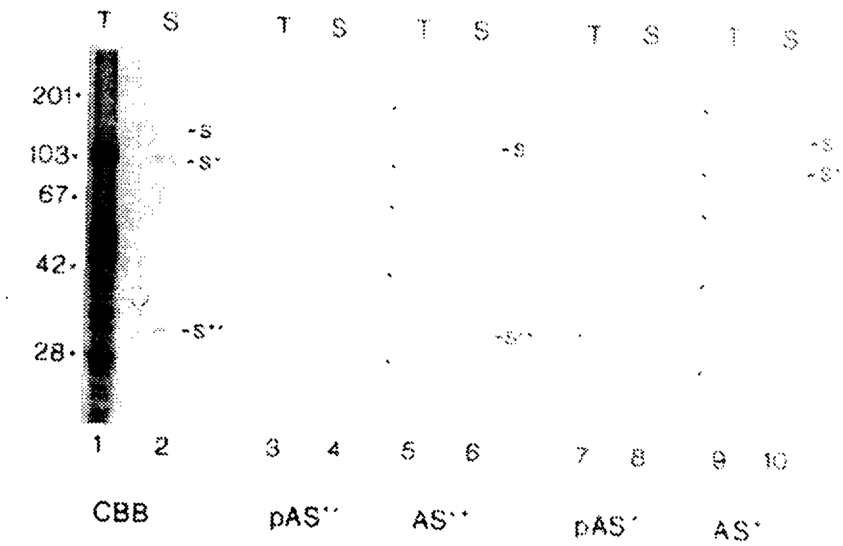


FIGURE 8

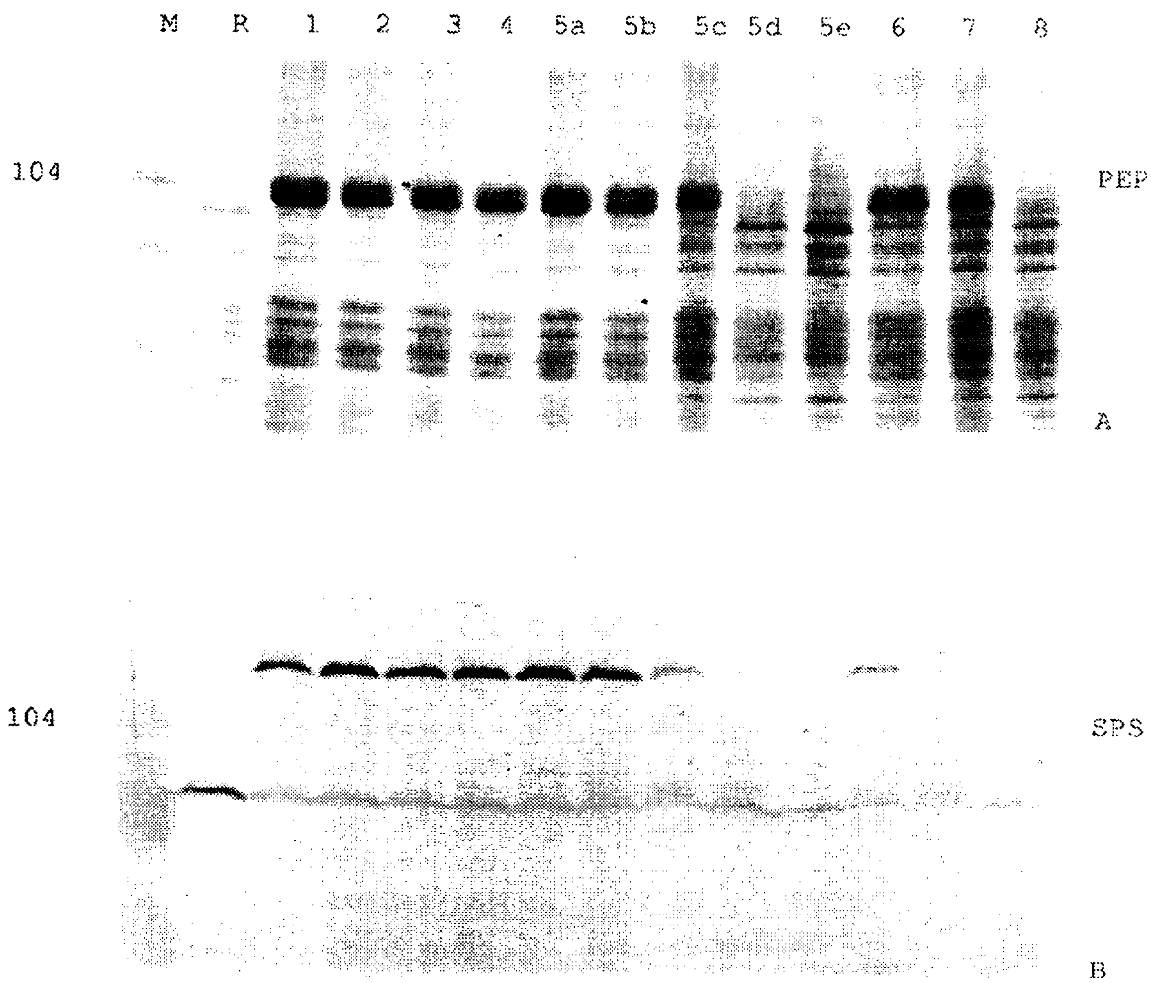


FIGURE 9